# EUROPEAN PATENT APPLICATION

(11) **EP 4 846 977 A2**
(43) Date of publication of application: **02.12.2026**
(21) Application number: 26212515.6
(22) Date of filing: 30.09.2021
(51) Int. Cl.: A61F 2/00

(54) **GLENOID IMPLANTS**

(30) Priority: 31.12.2020 US 202063132600 P
(62) Document number(s) of the earlier application(s) in accordance with Art. 76 EPC: 21916574.3 / 4 231 968
(71) Applicant: Howmedica Osteonics Corp., Mahwah, NJ 07430 (US)
(72) Inventor: GARGAC, Shawn Martin, Fort Wayne, 46818 (US); WOLFE, Alexander Paul, Fort Wayne, 46804 (US); MUTCHLER, Austin Wyatt, Warsaw, 46580 (US); COURTNEY, Robert, Pierceton, 46562 (US); IMM, Alan, Edgerton, 43517 (US); DASSONVILLE, Benjamin, 38660 Saint Hilaire du Touvet (FR); HENRY, Gilles, 38800 Le Pont de Claix (FR); STUMP, David R., Columbia City, 46725 (US); COULANGE, Vincent, 69003 Lyon (FR); GABORIT, Vincent, 38400 Saint Martin d'Hères (FR)
(74) Representative: Zimmermann & Partner Patentanwälte mbB

(57) **Abstract**

A glenoid implant, comprising: a body comprising: an articulation surface, a base surface on the opposite side of the body as the articulation surface, and a side wall extending between the articulation surface and the base surface; an undercut groove formed in the base surface of the body; a peripheral fixation feature provided along a periphery of the body, the peripheral fixation feature configured with a plurality of cutouts; and one or more additional fixation features extending from the base surface.

## Description

### FIELD OF DISCLOSURE

The present disclosure generally relates to glenoid implants for shoulder prosthesis.

### BACKGROUND

A shoulder prosthesis includes a glenoid implant intended to replace the glenoid cavity of the scapula and/or a humeral implant intended to replace the humeral head. The glenoid implant generally includes and articular body intended to articulate with the humeral head, and a fixation means to stabilize the articular body with respect to the scapula.

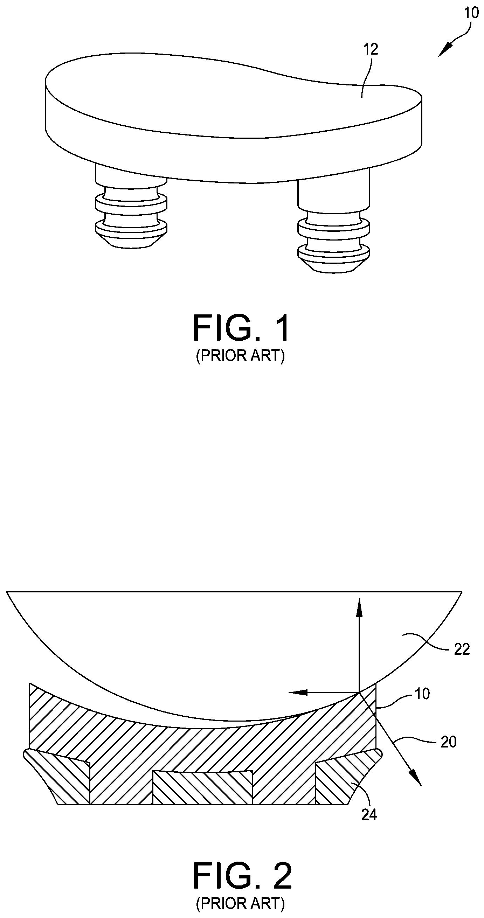

Optimum glenoid constraint may not be able to be achieved with a simple spherical surface. This principle is emphasized by the natural glenoid/labrum combination, which is not spherical and does not provide the same maximum constraint in all translation directions. Referring to FIG. 1, a currently available shoulder prosthesis glenoid component **10** has an articulation surface **12,** which is essentially defined by a spherical or dual radius, fully concave geometry. As such, prior art glenoid components do not take into account the differing levels of constraint required for different activities or the varying curvature of the natural glenoid.

Moreover, since currently available glenoid components have fully concave articulation surfaces, as the humeral head translates, the contact point between the head and glenoid will approach the edge of the glenoid. At a certain point, as illustrated in FIG. 2, a load vector **20** being applied to the glenoid component **10** by a humeral head **22** will no longer pass through the glenoid **24,** but will load the glenoid component **10** in an overhanging manner, significantly increasing loosening tendencies of the glenoid component **10.**

Typically, the glenoid prosthetic components are provided with one or more pegs or one or more keels on the side opposite from the articulation surface **12.** The pegs or keels are inserted into mating holes prepared in the glenoid cavity of the scapular neck. The pegs or keels are affixed to the scapular neck using bone cement.

Many prior art glenoid components are onlay design. In recent years, studies have found that inlay designs may offer improved stability of the glenoid component and reduce glenoid component loosening that are common in total shoulder arthroplasty. With growing interest in inlay designs for glenoid components, there is a need for new and improved inlay and/or onlay glenoid components.

### SUMMARY

Provided herein are various embodiments of glenoid implants that provide a replacement articulation surface of a glenoid in a shoulder. Many of the embodiments of the disclosed glenoid implants are inlay style implants with various fixation features. The implanted inlay implants will sit inside a reamed/drilled cavity in the glenoid such that the face opposing the articulation surface is positioned below the glenoid face. Some of the glenoid designs shown may be used as inlay or onlay implant.

### BRIEF DESCRIPTION OF THE DRAWINGS

The various embodiments of the inventive hydrogel implant of the present disclosure will be described in more detail in conjunction with the following drawing figures. The structures in the drawing figures are illustrated schematically and are not intended to show actual dimensions.
FIG. 1 is a perspective view of a prior art glenoid component with a spherical articulation surface and standard pegs.
FIG. 2 shows a side cross-sectional view of an illustration of an overhanging load on the prior art glenoid component.
FIG. 3 shows a glenoid prepared with a ring-shaped trough in which a ring-shaped glenoid implant according to the present disclosure is received.
FIG. 4 shows a fully seated ring-shaped glenoid implant of the present disclosure.
FIG. 5 shows a cross-sectional view of the fully seated ring-shaped glenoid implant.
FIG. 6 shows a cross-sectional view of the ring-shaped glenoid implant that is engaging a prosthetic humeral head.
FIG. 7 shows an embodiment of the ring-shaped glenoid implant comprising pegs along the bottom surface of the implant.
FIG. 8 shows another embodiment of the ring-shaped glenoid implant that is configured for cementless fixation into the glenoid.
FIG. 9 is a drill guide for identifying a center of a glenoid.
FIG. 10 is a perspective view of the drill guide of FIG. 9 positioned on a glenoid.
FIGS. 11-13 are illustrations showing the procedure for cutting a trough into the glenoid using a reamer.
FIG. 14 is an illustration of an example of a reamer for cutting a trough into the glenoid.
FIG. 15 is an illustration of a drill guide according to an embodiment of the present disclosure.
FIG. 16 is an illustration of a glenoid that has been prepared with a trough and blind holes for receiving the ring-shaped glenoid implant of FIG. 7.
FIG. 17 is an illustration of a glenoid implant according to another embodiment that is in an implanted state.
FIG. 18 is a side view illustration of the glenoid implant of FIG. 17.
FIG. 19 is an isometric view illustration showing the anchor surface of the glenoid implant of FIGS. 17-18.
FIG. 20-33 are illustrations showing the procedure for preparing a glenoid for the glenoid implant of FIGS. 17-19.
FIG. 34A is an isometric illustration of a glenoid implant according to another embodiment showing the articulation surface of the implant.
FIG. 34B is an isometric illustration of the glenoid implant of FIG. 34A showing the anchor surface of the implant.
FIGS. 35A-36B are a series of illustrations showing the procedure for preparing a glenoid for the glenoid implant of FIGS. 34A-34B.
FIGS. 37A-37B are illustrations showing a glenoid implant according to another embodiment.
FIG. 37C is a cross-sectional view of the glenoid implant of FIGS. 37A-37B.
FIGS. 37D-37F are illustrations showing an example of a procedure for preparing a glenoid to receive the glenoid implant of FIGS. 37A-37B.
FIGS. 37G-37I are illustrations showing a procedure for implanting the implant of FIGS. 37A-37B.
FIG. 38A-38B are illustrations showing a glenoid implant according to another embodiment.
FIG. 38C-38E are illustrations showing a procedure for implanting the implant of FIGS. 38A-38B into a prepared glenoid.
FIGS. 39A-39B are illustrations showing a glenoid implant according to another embodiment in its pre-implant configuration.
FIG. 39C is a cross-sectional view of the glenoid implant of FIGS. 39A-39B.
FIG. 39D-39E are illustrations showing the glenoid implant of FIGS. 39A-39B in its implanted configuration.
FIG. 39F is a cross-sectional view of the glenoid implant of FIGS. 39D-39E.
FIGS. 39G-39H are illustrations showing a procedure for preparing a glenoid to receive the glenoid implant of FIGS. 39D-39E.
FIG. 39I is a cross-sectional view illustration of a 2-in-1 reamer used in the procedure shown in FIGS. 39G-39H.
FIGS. 39J is an illustration of the recess in a glenoid formed by the procedure shown in FIGS. 39G-39I.
FIGS. 39K-39P are illustrations showing a procedure for implanting the glenoid implant of FIGS. 39A-39B.
FIGS. 40A-40C are illustrations showing a glenoid implant according to another embodiment.
FIG. 40D is an illustration of a glenoid that is prepared with a recess to receive the glenoid implant of FIG. 40A.
FIG. 40E is an illustration of the glenoid implant of FIG. 40A seated in the glenoid.
FIGS. 40F-40G are illustrations of all-polymer glenoid implant according to another embodiment.
FIGS. 40H-40N, 40P, and 40Q are additional embodiments of the all-polymer glenoid implant.
FIG. 40R is an illustration of a glenoid that is prepared with a recess to receive the glenoid implant of FIG. 40J.
FIG. 40S is an illustration of the glenoid implant of FIG. 40J seated in the glenoid.
FIG. 40T is an illustration of the glenoid implant of FIG. 40I seated in the glenoid.
FIG. 40U is an illustration of a portion of the recess prepared in a glenoid to receive one of the all-polymer glenoid implants wherein the portion is configured to form an interference fit with the all-polymer glenoid implants.
FIGS. 41A-41F are illustrations of various embodiments of another glenoid implant according to the present disclosure.
FIG. 41G is a detailed illustration showing the interference fit between the glenoid and the glenoid implants shown in FIGS. 41A-41F.
FIGS. 42A-42D are illustrations of a metal-backed glenoid implant embodiment.
FIGS. 42E-42F are illustrations of the metal anchor component of the metal-backed glenoid implant of FIGS. 42A-42D.
FIGS. 42G-42H are illustrations of a glenoid prepared with a recess for receiving the metal-backed glenoid implant of FIGS. 42A-42C.
FIGS. 43A-43B are illustrations of a porous metal-backed glenoid implant embodiment.
FIG. 43C is an illustration of an exploded view of the porous metal-backed glenoid implant of FIGS. 43A-43B.
FIG. 43D is a cross-sectional view of the porous metal-backed glenoid implant of FIGS. 43A-43B.
FIG. 43E is a detailed view of the region A noted in FIG. 43D.
FIG. 43F is an illustration of a view of the porous metal-backed glenoid implant of FIGS. 43A-3B looking straight on to the articulation surface **1130** of the implant.
FIG. 43G is an illustration showing how various modular non-peripheral fixation features can be used in combination with the metal-backed glenoid implant of FISG. 43A-43B.
FIG. 43H is a cross-sectional view of an implant converted to reverse configuration. The tapered cylindrical feature would assemble with a glenosphere. Additionally, there can be screw holes that go through this cylindrical attachment.
FIG. 44A is an isometric view illustration of a glenoid implant employing a circular bone engagement rim according to another embodiment.
FIG. 44B is a side view of the glenoid implant of FIG. 44A.
FIG. 44C is a cross-sectional view of the glenoid implant of FIG. 44A.
FIG. 44D is an isometric view illustration of a glenoid implant employing a circular bone engagement rim according to another embodiment.
FIGS. 44E-44F are cross-sectional view illustrations of variations of the glenoid implant of FIG. 44A.
FIG. 45A is an isometric view illustration of a glenoid implant employing a circular bone engagement rim according to another embodiment.
FIG. 45B is a side view illustration of the glenoid implant of FIG. 45A.
FIG. 45C is an isometric view illustration of a glenoid implant employing a circular bone engagement rim according to another embodiment.
FIG. 45D is a side view illustration of the glenoid implant of FIG. 45C.
FIG. 45E is an illustration showing how a glenoid may be prepared with a recess for receiving the glenoid implants shown in FIGS. 44A and 45A.
FIG. 45F is an illustration showing the glenoid implant of FIG. 44A or 45A implanted in a glenoid.
FIGS. 45G-45H are illustrations showing examples of cutting instruments for preparing a glenoid for receiving the glenoid implants shown in FIGS. 44A and 45A.
FIG. 46A is an isometric view illustration of a glenoid implant employing a circular bone engagement rim according to another embodiment.
FIG. 46B is a cross-sectional view illustration of the glenoid implant of FIG. 46A.

### DETAILED DESCRIPTION

This description of the exemplary embodiments is intended to be read in connection with the accompanying drawings, which are to be considered part of the entire written description. The drawing figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures.

Provided herein are various improved glenoid implants that have articulation surface that is configured to engage with an anatomical humeral head or a humeral component of a shoulder replacement implant system. Therefore, references to "a humeral head" as used herein should be construed to include both an anatomical humeral head as well as implant humeral head.

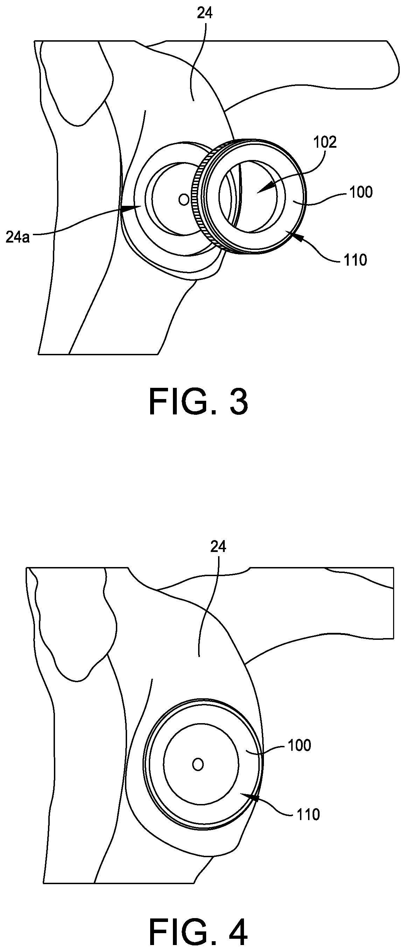

According to an embodiment illustrated in FIGS. 3-7, an inlay glenoid implant **100** is provided that has a ring-like structure. The ring-shaped glenoid implant **100** has a ring-shaped body **101** that comprises a hole **102.** The ring-shaped body **101** comprises an articulation surface **110** provided on one side and a base surface **101a** provided on the opposite side. The hole **102** extends completely through the implant **100** from the articulation surface **110** to the base surface **101a.** When implanted into a glenoid **24,** the articulation surface **110** faces outward from the glenoid **24** and replaces the natural articulation surface of the glenoid cavity. The articulation surface **110** is configured to engage with a humeral head.

The ring shape of this glenoid implant allows stabilization of the shoulder while minimizing bone reaming/removal. Minimizing bone reaming/removal can be beneficial for healing of the surgical site. The ring shape having a hole in the center allows the glenoid to support differing mating component curvatures while maintaining a continuous ring of contact. This differs from a conventional articulation surface that has a theoretical single point contact (not accounting for material deformation). Additionally, this nature of the ring contact offers a self-centering force to the mating humeral spherical head. Self-centering is good to help keep the humeral head from moving off the glenoid.

The hole **102** can be any desired shape and any size. For example, in some embodiments, the hole **102** can have a circular shaped outline as shown. In some other embodiments, the hole can be configured to have a polygon shaped outline. The polygon can be a regular or an irregular polygon. In some embodiments, the hole **102** can be configured as a patient specific irregular shaped hole that is customized to match the shape of an area of the patient's glenoid that is desirable to keep intact so that the ring-shaped implant 100 surrounds the area being saved.

The curvature of the articulation surface **110** can be configured to have any desired contour. In some embodiments, the curvature of the articulation surface **110** can be spherical.

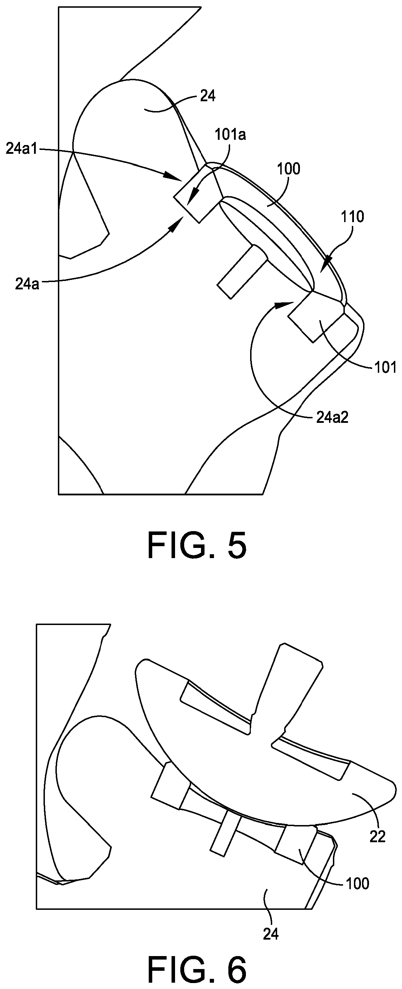

Referring to FIG. 3, the glenoid **24** is prepared with a ring-shaped recess **24a** in which the ring-shaped glenoid implant **100** gets positioned. FIG. 4 shows the ring-shaped glenoid implant **100** that is seated in the ring-shaped recess **24a.** FIG. 5 shows a cross-sectional view of the seated ring-shaped glenoid implant **100.** The bottom surface **101a** of the ring-shaped body **101** is in contact with the bottom surface of the ring-shaped recess **24a.** The cross-section view in FIG. 6 shows a humeral head **22** engaging the articulation surface **110** of the ring-shaped glenoid implant **100.**

The corresponding surfaces of the ring-shaped body **101** and the ring-shaped recess **24a** are configured to provide intimate contact and enable the glenoid implant **100** to be securely seated in the recess **24a.** The base surface **101a** of the ring-shaped body **101** and the bottom surface of the ring-shaped trough **24a** are contoured to match each other's contour to ensure that the two surfaces are intimately in contact when the glenoid implant **100** is inserted into the ring-shaped recess **24a.** In the illustrated example, the base surface **101a** and the bottom of the ring-shaped recess **24a** are flat. In some embodiments, however, the base surface **101a** can be concave, convex, or flat and the bottom surface of the ring-shaped trough **24a** has a complementary contour. Similarly, the inner surface **105** and the outer surface **103** of the ring-shaped glenoid implant **100** are substantially orthogonal to the base surface **101a.** The ring-shaped recess **24a** comprises an inner surface **24a2** and an outer surface **24a1** that are substantially orthogonal to the bottom surface of the recess **24a.** (See FIG. 5). When the glenoid implant **100** is inserted into the ring-shaped recess **24a** for implantation, the corresponding surfaces of the glenoid implant **100** and the ring-shaped recess **24a** come in contact with each other and securely hold the glenoid implant **100** in place.

In some embodiments, the ring-shaped glenoid implant **100** can be formed in whole or in part, particularly the portion that forms the articulation surface **110,** of a synthetic material, such as, for example, polyethylene (e.g. ultra-high-molecular-weight polyethylene (UHMWPE)), polyether ether ketone (PEEK), etc. All references to UHMWPE herein includes all variants of UHMWPE in orthopedic application such as vitamin E diffused UHMWPE. In a preferred embodiment, the ring-shaped glenoid implant **100** can be formed in whole or in part, particularly the portion that forms the articulation surface **110,** of hydrogel material.

The hydrogel material referred to herein refers to a three-dimensional solid resulting from cross-linked hydrophilic polymer chains formed of polyvinyl alcohol (PVA). The hydrogel material can comprise one or more other materials in addition to PVA, such as, for example, other hydrogels, other polymeric materials, additives, and/or the like. In some embodiments, the PVA content of the hydrogel in the implants disclosed herein can be about 40% by weight. The PVA content of the hydrogel can range from about 10% by weight to about 80% by weight, as appropriate for particular application.

The hydrogel can comprise water, saline, other liquids, combinations thereof, and/or the like. In some embodiments, saline may be preferred over water, because, under certain circumstances, saline can help maintain osmotic balance with surrounding anatomical tissues following implantation. The exact composition of the hydrogel component in an implant can be selected for optimal performance in a particular application to achieve the desired or required strength, load bearing capacity, compressibility, flexibility, longevity, durability, resilience, coefficient of friction, and/or other properties and characteristics.

In some embodiments, such hydrogel portion(s) of the ring-shaped glenoid implant 100 and all other embodiments of glenoid implants disclosed herein can be formulated for drug delivery and/or is seeded with growth factors and/or cells. In such embodiments, the hydrogel component can comprise one or more of the following: chondrocytes, growth factors, bone morphogenetic proteins, collagen, hyaluronic acid, nucleic acids, and stem cells. Such factors and/or any other materials included in the implants can help facilitate and/or promote long-term fixation of the implants at the joint site.

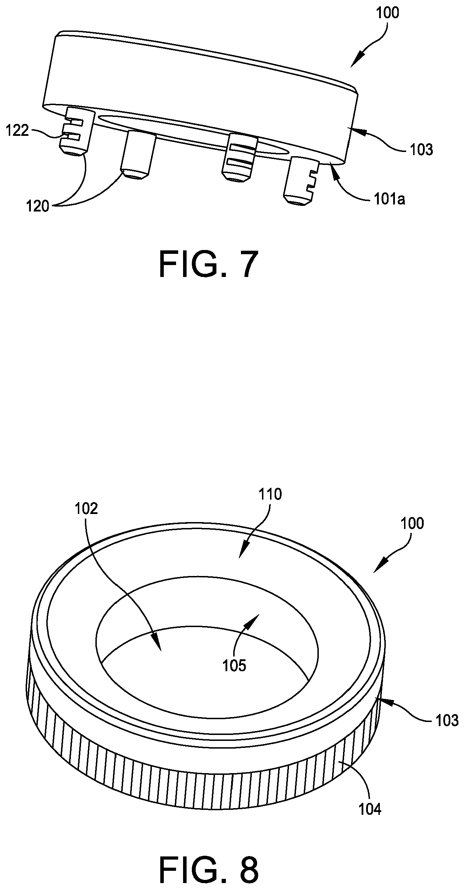

The ring-shaped glenoid implant can be affixed into the prepared glenoid **24** using a variety of methods. FIG. 7 shows an embodiment of the ring-shaped glenoid implant **100** can comprise one or more pegs **120** along the base surface **101a** for securing the implant into the glenoid **24.** Each of the pegs **120** can comprise one or more slots **122** for accommodating bone cement to secure the implant **100** in the bone **24.** To use this embodiment of the ring-shaped glenoid implant the glenoid **24** would need to be prepared with corresponding holes for the pegs **120.** The procedure for preparing the bone will be described below.

In FIG. 8, another embodiment of the ring-shaped glenoid implant **100** is shown that is configured for cementless fixation into the glenoid **24.** In this embodiment, the glenoid implant **100** comprises at least a portion of the outer surface **103** that is coated with a porous trabecular metallic material **104,** such as ADVANCE^{®} BIOFOAM^{™} of Wright Medial Technology, Inc., for bone ingrowth into the glenoid implant. The structure of the coated trabecular metallic material resembles that of trabecular bone. In some embodiments, at least some portion(s) of the interior surface **105** of the ring-shaped glenoid implant **100** can also be coated with a trabecular metallic material.

In some embodiments of the ring-shaped glenoid implant **100** that is configured for cementless fixation, the bulk of the ring-shaped glenoid implant **100** that includes the bone-engaging base surface **101a** can be formed of the porous trabecular metallic material and the articulation surface portion can be made of a hydrogel that is bonded to the porous trabecular material.

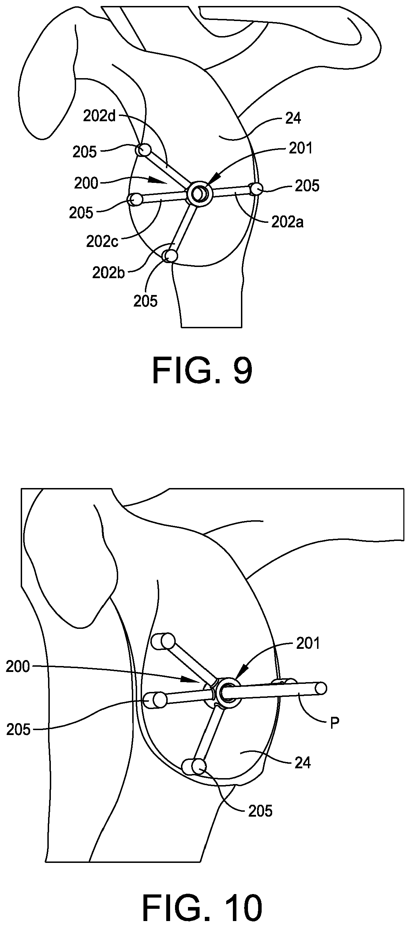

Referring to FIGS. 9 through 17, a corresponding procedure for preparing the glenoid **24** for receiving a ring-shaped glenoid implant **100** is disclosed. Shown in FIG. 9 is a drill guide **200** for identifying a center of the glenoid **24** that is positioned over the glenoid **24.** The drill guide **200** comprises a guide hole **201** and a plurality of arms **202a, 202b, 202c,** and **202d** that radially extend from the guide hole **201** orthogonal to the central axis of the guide hole **201.** Each of the arms **202a-202d** are provided with an edge guide **205** at the arm's terminal end. As can be seen in the perspective view in FIG. 10, each of the edge guides **205** extend orthogonal to their respective arms and they are configured to be used to straddle the perimeter edge of the glenoid **24** as illustrated. As shown in FIG. 9, in the preferred embodiment of the drill guide **200,** two of the arms **202a** and **202c** extend out from the guide hole **201** at 180° apart from each other. The remaining two arms **202b** and **202d** are positioned straddling the arm **202c.** Because all of the arms extend out from the guide hole **201** orthogonal to the central axis of the guide hole **201,** the arms are in the same plane.

The arrangement of the arms and their edge guides **205** allow the edge guides **205** to fit around the perimeter of the glenoid **24** as shown so that the guide hole **201** automatically locates the geometric center. To accommodate different size glenoid among the patients, the drill guide **200** can be provided in a variety of graduated sizes.

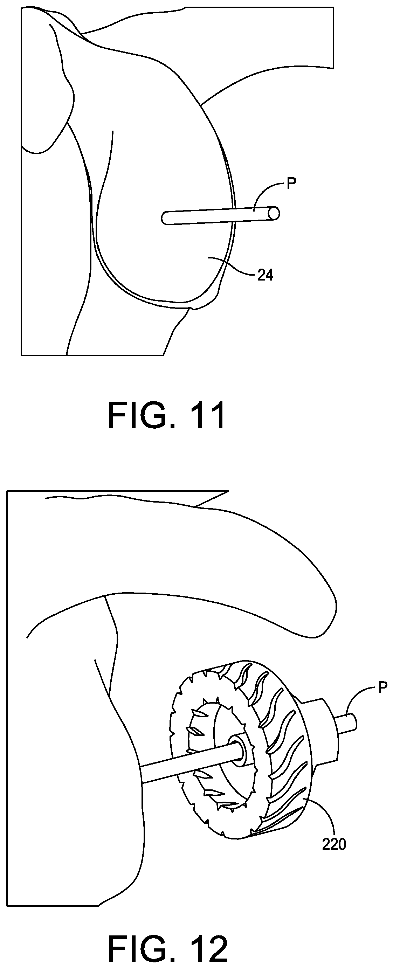

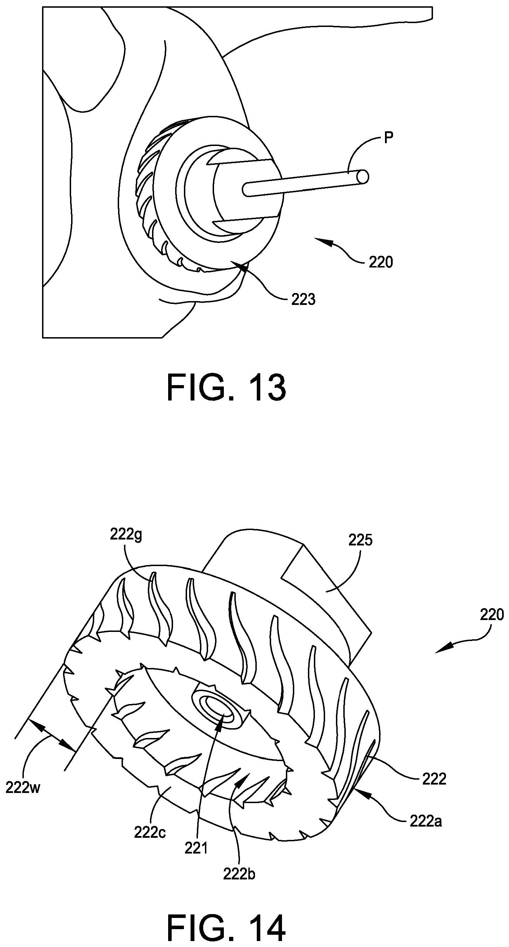

After the appropriately sized drill guide **200** is placed over the glenoid **24** as shown in FIG. 9, a hole is drilled into the glenoid **24** via the guide hole **201.** Then, a pin **P** is inserted into the drilled hole as shown in FIG. 10. Referring to FIGS. 11 and 13, once the pin **P** is in place, the drill guide **200** is removed and a reamer **220** is used to carve the ring-shaped trough **24a** into the glenoid **24.**

The placement of the drill guide **200** on the glenoid **24** can be accomplished visually or with the aid of Wright Medical Technology's Blueprint^{™} 3D surgical planning system. Furthermore, the drill guide **200** can be a patient specific instrument fabricated using the Blueprint^{™} system.

As shown in FIGS. 13 and 14, the reamer **220** is generally shaped like a bell saw and comprises a mandrel portion **223** and a cylindrical blade portion **222.** The cylindrical blade portion **222** has a cutting end **222c** that has a width **222w** that creates trough **24a.** The cylindrical blade portion **222** has an outer wall **222a** and an inner wall **222b.** The two circular edges defined by the cutting end **222c** and the outer and inner walls **222a, 222b** form the cutting edges. The outer and inner walls **222a, 222b** can further comprise grooves **222g** that aids in the cutting action as well as expulsion of the bone cutting debris during the reaming procedure. The shape and dimensions of the grooves **222g** can be varied to optimize the reaming efficiency of the reamer **220.** Provided at the center of the mandrel portion **223** is a hole **221** so that the reamer **220** can be placed over the pin **P.** The mandrel portion **223** comprises a driving tool engaging portion **225.** The driving tool engaging portion **225** is configured to engage a driving tool, such as a surgical hand drill, that can turn the reamer **220** for the reaming action.

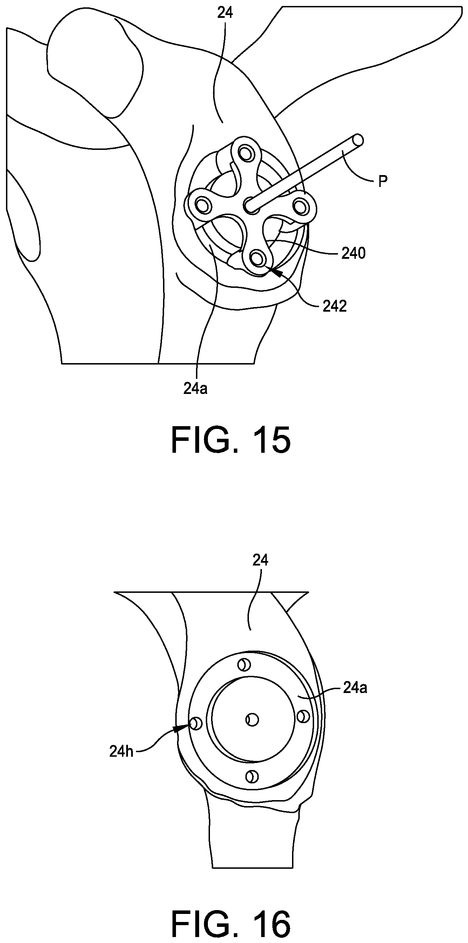

Where the glenoid implant **100** is the embodiment shown in FIG. 7 that has a plurality of pegs **120** for securing the implant into the glenoid **24,** a drill guide **240** shown in FIG. 15 can be used to prepare the blind holes for receiving the pegs **120** in the bottom of the trough **24a.** The drill guide **240** is configured to be slipped over the pin **P** so that the drill guide **240** is in alignment with the trough **24a.** FIG. 16 is an illustration of a glenoid **24** that has been prepared with a trough **24a** and blind holes **24h** for receiving the glenoid implant **100** embodiment shown in FIG. 7.

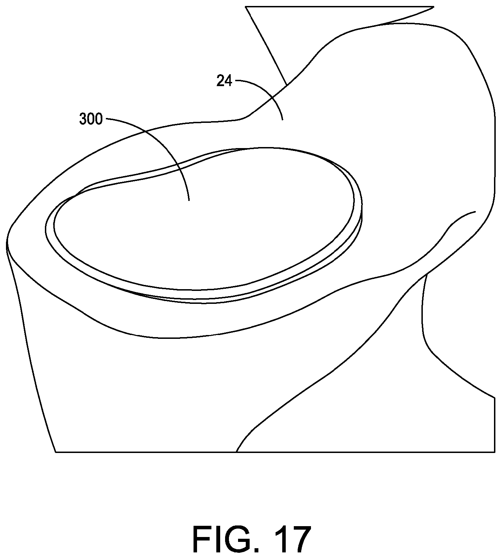

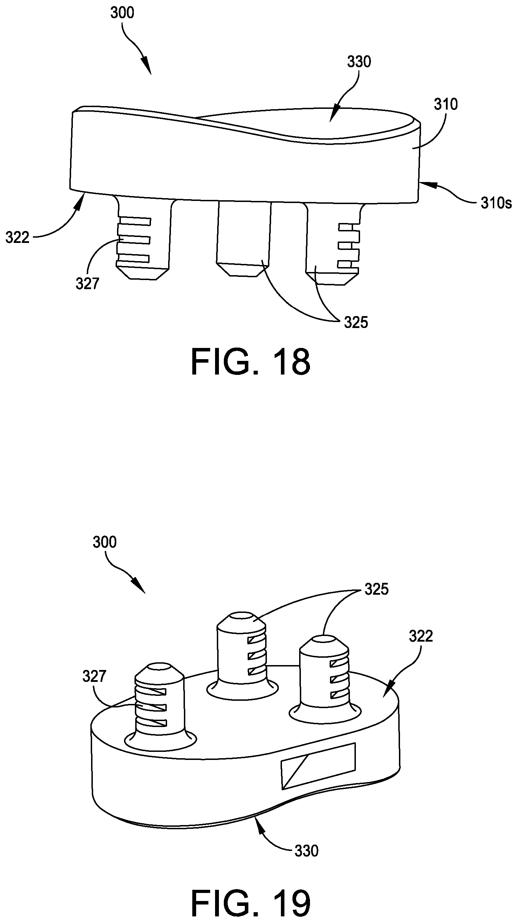

Referring to FIGS. 17 - 21, an embodiment of a glenoid implant **300** that is configured to be implanted into a glenoid in an inlay configuration is disclosed. FIG. 17 shows the glenoid implant **300** in an implanted state in a glenoid **24.** FIG. 18 is a side view of an embodiment of the glenoid implant **300.** FIG. 19 is an isometric view of the glenoid implant **300.** The glenoid implant **300** comprises a body **310** comprising an articulation surface **330** and an anchor surface **322** on the opposite side. The glenoid implant **300** can be configured to have a shape that maximizes the articulation surface of the glenoid **24** that is replaced by the glenoid implant **300.** The articulation surface **330** is contoured to replicate the anatomical articulation surface of the glenoid **24.** In some embodiments, the anchor surface **322** comprises one or more fixation features such as posts, finned anchors, or keel, etc. extending therefrom. In the illustrate example shown, three anchors **325** extend from the anchor surface **322.**

As shown in FIG. 17, the glenoid implant **300** is sized and shaped to be implanted into a glenoid **24** that is prepared with a recess to receive the glenoid implant **300.** Inlay glenoid implant such as the glenoid implant **300** and the ring-shaped glenoid implant **100** are smaller than the full surface of the glenoid **24.** Therefore, inlay configuration allows replacing just the defective or damaged portion of the glenoid's articulation surface and minimize disturbing the native glenoid bone material.

In some embodiments, each of the one or more anchors **325** can comprise one or more bone cement pockets **327.** When the glenoid implant **300** is being secured into the corresponding recess prepared in the glenoid **24,** bone cement can be used to enhance the securement of the implant. Each of the pockets **327** in the anchors **325** holds an amount of bone cement and assist in the securement of the glenoid implant **300.**

In some embodiments, the glenoid implant **300** can be formed in whole or in part, particularly the portion that forms the articulation surface **110,** of synthetic material, such as, for example, polyethylene (e.g. ultra-high-molecular-weight polyethylene (UHMWPE)), polyether ether ketone (PEEK), etc. In a preferred embodiment, the articulation surface **330** can be formed of the hydrogel material mentioned above.

In some embodiments, the glenoid implant **300** can be formed of a suitable surgical grade metal or metal alloy. Some examples are cobalt-chrome alloys and titanium alloys. When the glenoid implant **300** is made of a metal or metal alloy, the body **310** of the implant can be thinner because of the added stiffness and strength provided by metal. In the metal embodiments of the glenoid implant **300,** the anchor surface **322** can be coated with porous trabecular metal coating mentioned above to promote ingrowth of bone tissue after the implant **300** is implanted. In some embodiments, portions of sidewall **310s** (See FIG. 18) of the implant **300** can also be coated with porous trabecular metal coating.

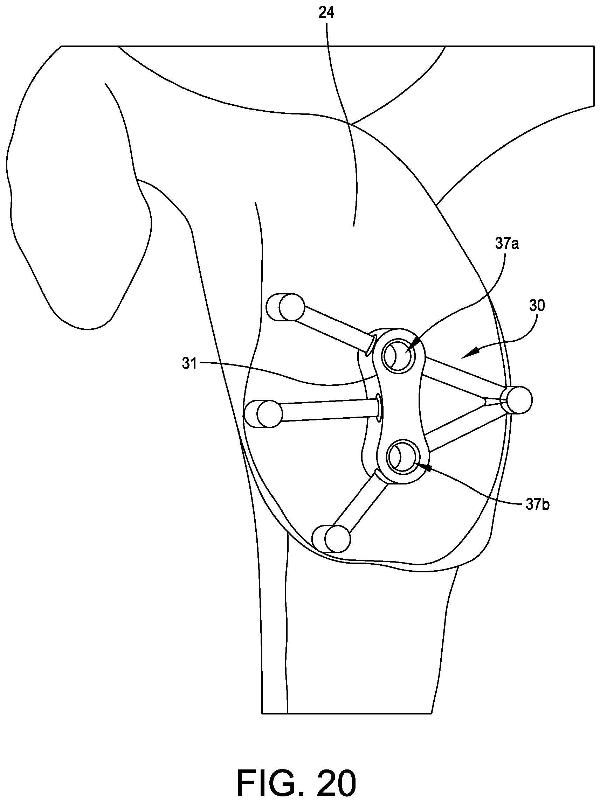

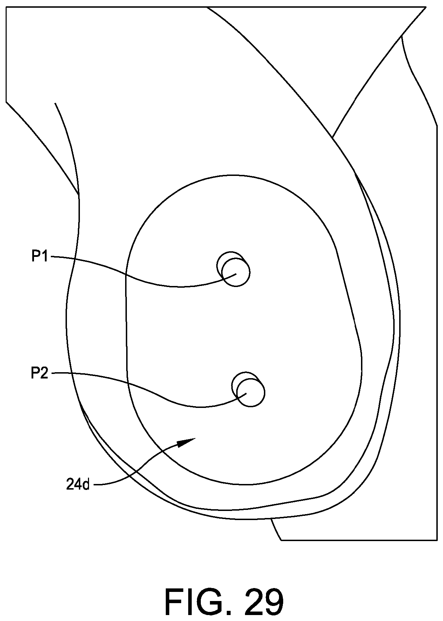

Referring to FIGS. 20-31, a corresponding procedure for preparing the glenoid **24** for receiving a glenoid implant **300** is disclosed. First, a drill guide **30** is placed on the glenoid **24** to locate the locations for drilling two blind holes for guide pins. The placement of the drill guide **30** on the glenoid **24** can be accomplished visually or with the aid of Wright Medical Technology's Blueprint^{™} 3D surgical planning system. In some embodiments, the drill guide **30** can be a patient specific instrument fabricated using the Blueprint^{™} 3D surgical planning system.

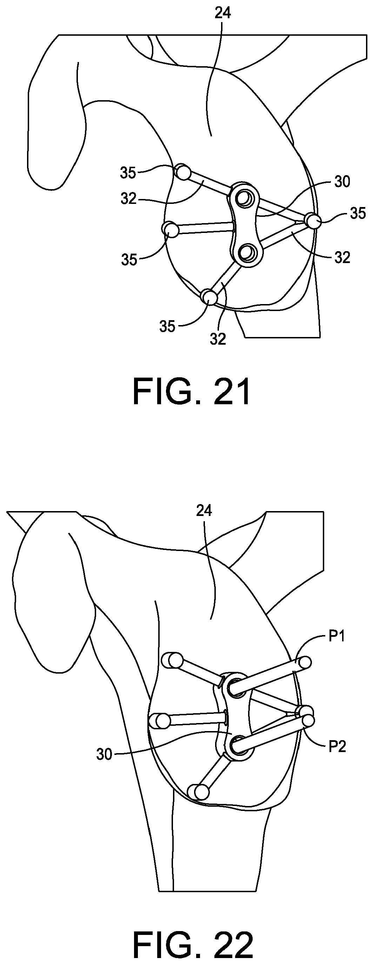

The drill guide **30** comprises a main body **31** that is provided with two drill guide holes **37a, 37b.** The main body **31** comprises a plurality of arms **32** that extend outward from the main body **31** that terminate with edge guides **35** provided at the ends of the arms **32.** Each of the edge guides **35** extend orthogonal to their respective arms and they are configured to be used to straddle the perimeter edge of the glenoid **24** as illustrated in FIGS. 20 and 21. The drill guide **30** can be provided in a variety of sizes to accommodate different size glenoid in different patients. Positioning an appropriately sized drill guide **30** on a glenoid **24** will position the two drill guide holes **37** in the desired position. Once the drill guide **30** is in the desired position, two blind holes are drilled into the glenoid **24** using the guide holes **37.** Next, two guide pins **P1, P2** are placed into the drilled blind holes in the glenoid **24** and the drill guide **30** is removed. See FIGS. 22-23.

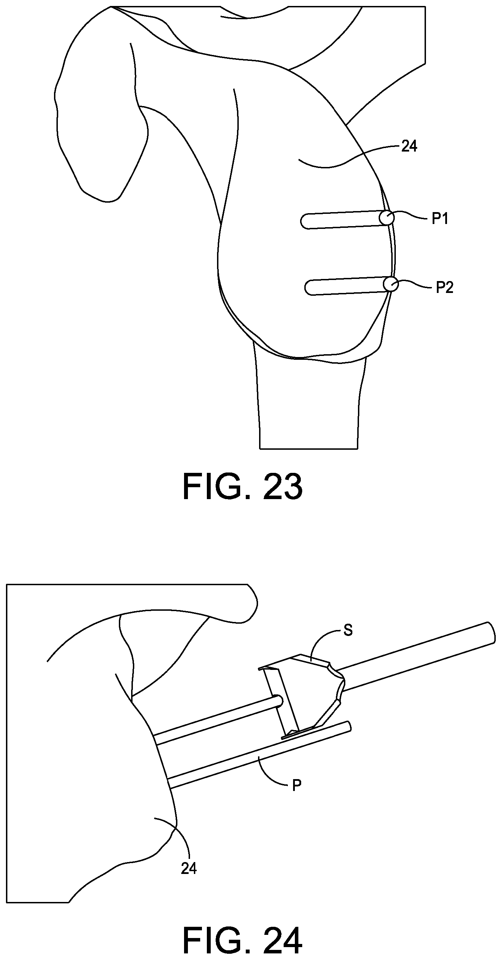

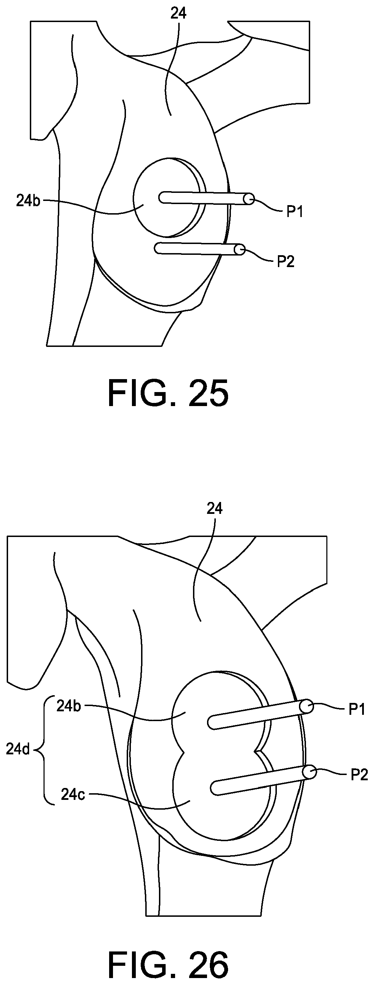

Next, a series of bone reaming procedure is carried out to form an appropriately shaped recess in the glenoid **24** to receive the glenoid implant **300.** Referring to FIG. 24, a spade drill **S,** guided by the pins **P1, P2,** is used to form two, wide and shallow, blind holes (e.g. a recess). FIG. 25 shows first shallow, blind hole **24b** thus formed. In the illustrated example, the guide pin **P1** was used first to form the first blind hole **24b** but the other guide pin **P2** could have been used first. This procedure is repeated over the guide pin **P2** to form a second shallow blind hole **24c** as shown in FIG. 26. The two shallow blind holes **24b** and **24c** overlap as shown forming a single shallow recess **24d.** The depth of the shallow blind holes are determined to accommodate the thickness of the glenoid implant **300.**

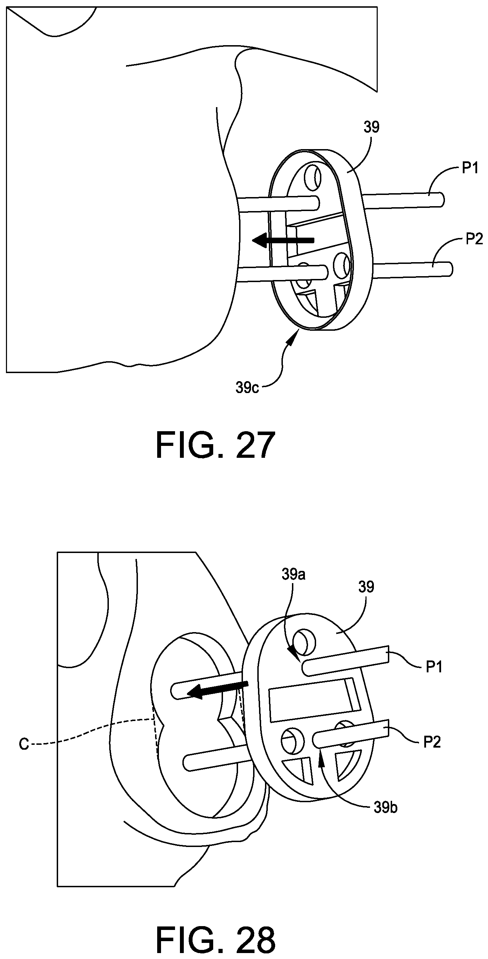

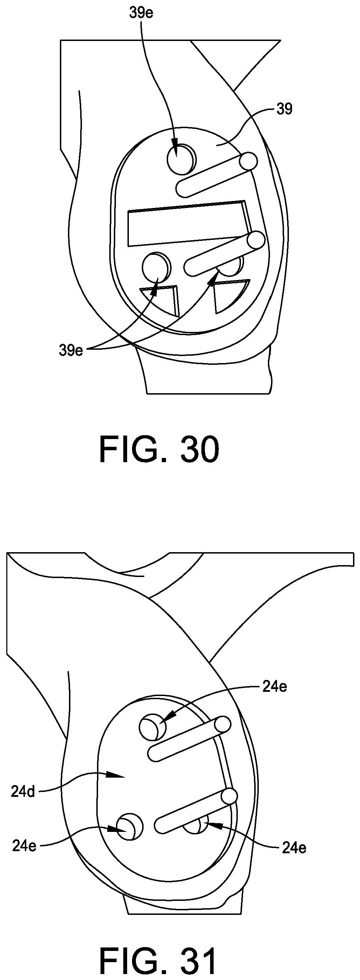

Referring to FIGS. 27-29, a cookie cutter type bone cutting device **39** is applied to create the final outline for the shallow recess **24d** to receive the glenoid implant **300.** The bone cutting device **39** has a cutting edge **39c** that is shaped to cut the bone between the two shallow blind holes **24b, 24c** along the dotted line **C** shown in FIG. 28. This removes the excess bone material between the two shallow blind holes **24b, 24c** and form a recess **24d** in the glenoid **24** having an outline of the glenoid implant **300** as shown in FIG. **30****.** To help guide and align the cookie cutter type bone cutting device **39,** the bone cutting device can be provided with two guide holes **39a** and **39b** that are appropriately sized and located on the bone cutting device **39** to be slid over the pins **P1, P2.** Once the bone cutting device **39** is in place with the cutting edge **39c** contacting the intended cutting line **C,** the bone cutting device **39** can be tapped down to cut into the bone.

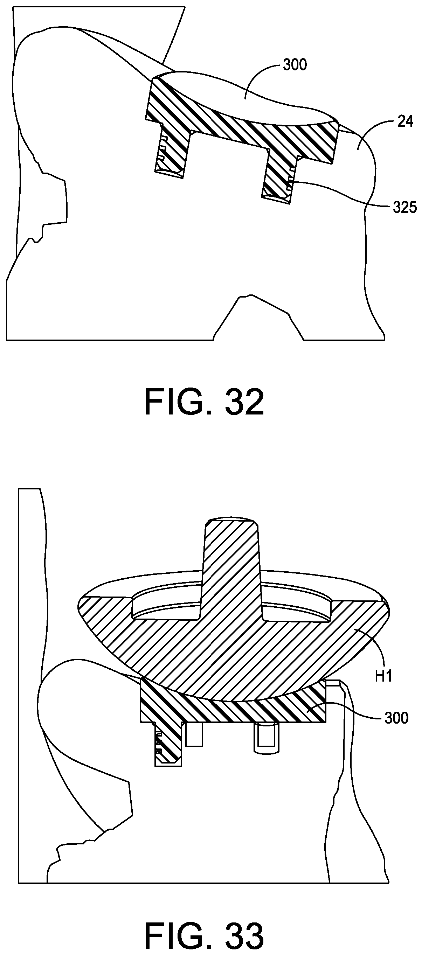

Referring to FIG. 30, the bone cutting device **39** can further comprise additional drill guide holes **39e** that can be used to drill blind holes for receiving the anchors **325** that can be provided on the glenoid implant **300.** FIG. 32 is a sectional view of the implanted glenoid implant **300.** FIG. 33 shows an example of a humeral head implant **H1** interacting with the articulation surface of the glenoid implant **300.**

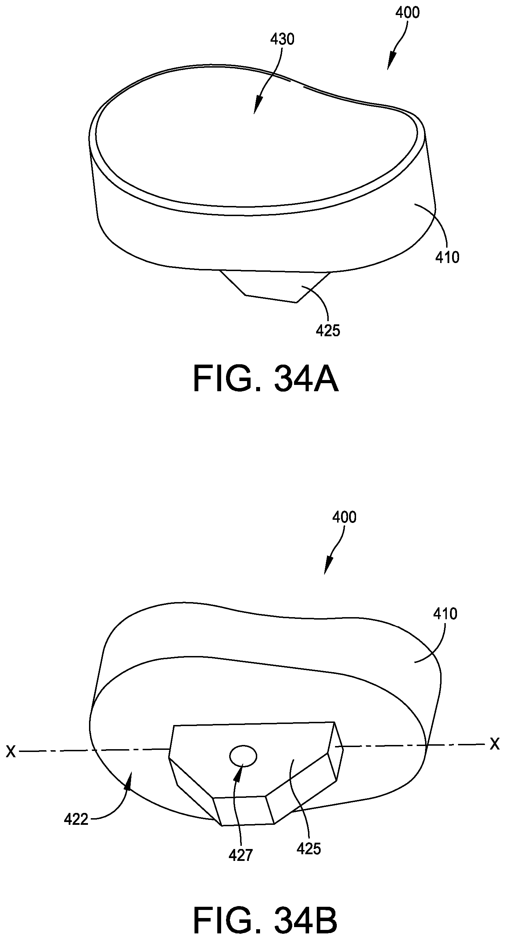

Referring to FIGS. 34A-34B, another embodiment of a glenoid implant **400** that is configured to be implanted into a glenoid in an inlay configuration is disclosed. Similar to the glenoid implant **300,** the implant **400** can be configured with a shape that maximizes the articulation surface area of the glenoid **24** that is replaced by the implant **400.** FIG. 34A shows an isometric view of the top side, i.e. an articulation surface **430** side, of the glenoid implant **400.** FIG. 34B is an isometric view of the glenoid implant **400** from the bottom side. The glenoid implant **400** comprises a body **410** having the articulation surface **430** and an anchor surface **422** on the opposite side. The articulation surface **430** s contoured to emulate the natural articulation surface of the glenoid **24.** The articulation surface **430** includes a concave profile that is intended to cooperate in the anatomical manner with the humeral head whether it be a natural one or a prosthetic one. The anchoring surface **422** can be coated with a porous trabecular metallic material such as Wright Medical Technology's ADAPTIS^{™} that can promote bone tissue ingrowth to enhance bonding of the glenoid implant **400** to glenoid after the implant is implanted to a prepared glenoid.

Extending from the anchor surface **422** is an anchoring keel **425.** The anchoring keel **425** is configured to securely anchor the implant **400** in the glenoid **24** in an inlay configuration and to minimize or prevent rocking of the implant **400** after it is implanted. The keel **425** extends along longitudinal axis X-X. The keel **425** preferably includes a longitudinal dimension or length that is greater than its transverse dimension or width. The keel **425** can have a structure of an elongated ridge or upstanding structure attached to the glenoid implant's body **410** with a length oriented along a longitudinal axis of the glenoid implant **400.** The keel **425** can have a length along the longitudinal axis X-X that is greater than, less than, or the same as the body **410** of the glenoid implant **400.** The keel **425** can have generally planar sides, which can include a variety of protrusions, recesses, anchor members, and holes.

In the illustrated example shown in FIG. 34B, the keel **425** includes a transverse hole **427,** allowing the creation of a cement bridge for fixing the implant **400,** if it is cemented. If the glenoid implant **400** is not cemented, the hole **427** is likely to allow the creation of a bone bridge. In some embodiments, the hole **427** may be used to receive one or more fasteners, such as bone screws.

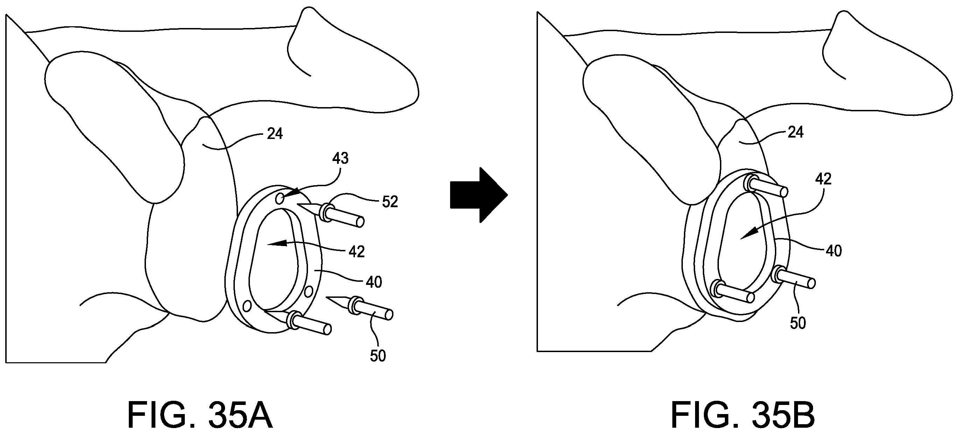

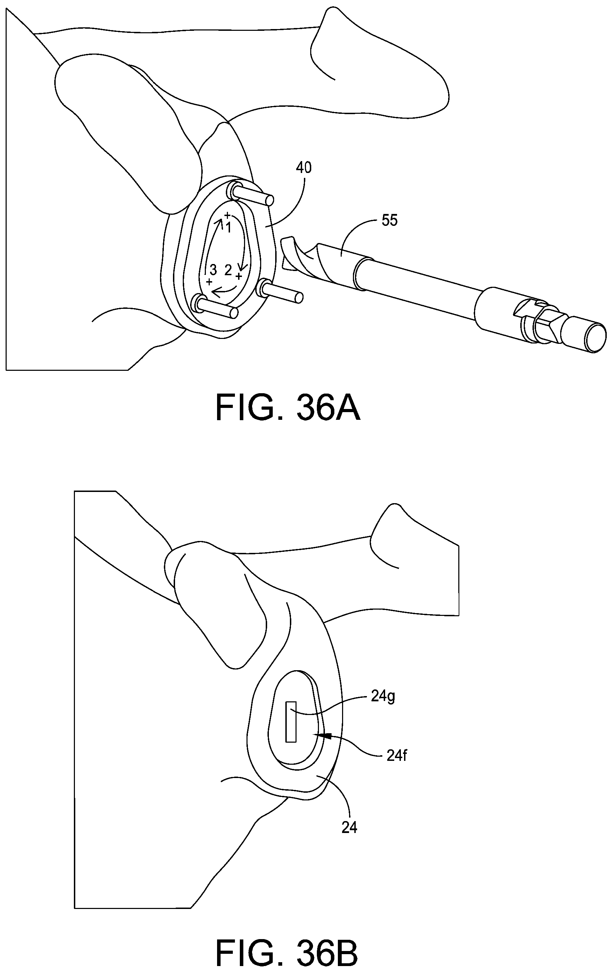

The process of preparing the glenoid **24** to receive the glenoid implant **400** will now be described in conjunction with FIGS. 35A-36B. The process involves using a milling guide **40** to remove a portion of the glenoid **24** from the articulation surface of the glenoid **24** to form a shallow recess that can receive the implant **400.** The milling guide **40** has a ring-like structure with an opening **42** in the center. Referring to FIG. 36A, after the milling guide **40** is placed on the glenoid **24** at a desired location, the opening **42** allows access to the area of the glenoid **24** for removal using a reamer bit **55.** As shown in the caption for FIG. 36A, an arbitrary example pathway for the milling operation using a reamer bit can be as shown by the arrows indicated inside the opening **42.** The direction of the movement for the reamer bit can be in any direction as long as the reamer is maintained within the opening **42.** If some bone material remains at the site after using the reamer bit, especially in the center of the opening **42,** the bone material can be removed with a rongeur drill bit to finish the preparation of the bone.

Referring to FIG. 36B, the milling operation results in a shallow recess **24f** formed in the glenoid **24** that is shaped to receive the glenoid implant **400.** The slot **24g** for the keel can be formed using a punch tool.

The shape of the opening **42** can be any desired shape. It can be circular, oval, piriform, etc. The back side of the milling guide **40** that comes in contact with the glenoid can be flat that can be used universally on all patients or the surface can be pre-formed with a customized surface that has a contour that is patient specific. The milling guide **40** also comprises a plurality of holes **43** provided on its ring-like structure for accommodating fixation pins/tacks **50** to temporarily affix the milling guide **40** in place during the subsequent reaming procedure. In the illustrated example, the milling guide **40** has three such holes **43.** The pins **50** comprise a shoulder **52** in the middle of its length that has a diameter larger than the holes **43** so that after the pins **50** are in place as shown in FIG. 35B, the milling guide **40** cannot slide up the pins **50.**

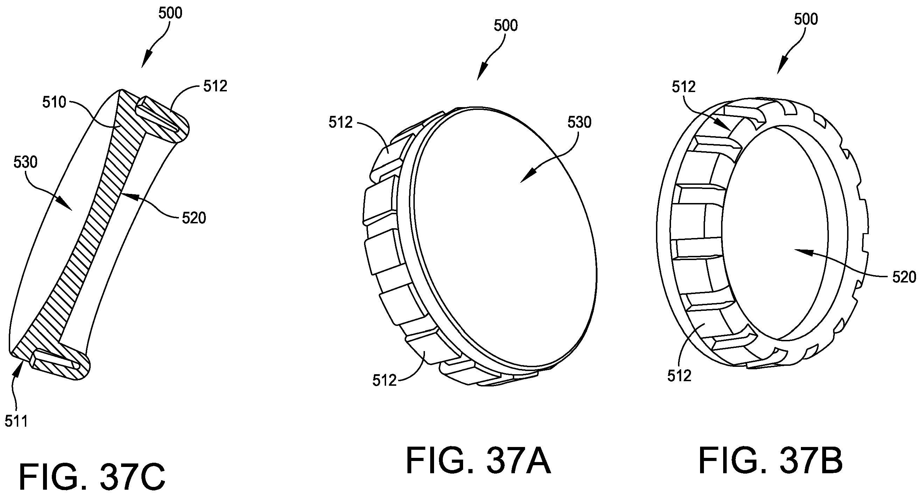

Referring to FIGS. 37A-37C, a glenoid implant **500** according to another embodiment is disclosed. The glenoid implant **500** is configured to be securely press fitted into a recess prepared into a glenoid to replace damaged natural articulation surface. The glenoid implant **500** comprises a circular body **510** having an articulation surface **530** on one side, an anchoring surface **520** on the opposite side, and an annular side wall **511** extending around the periphery of the circular body **510.** The articulation surface **530** is contoured with a generally concave surface that emulates the natural articulation surface of a glenoid. The articulation surface **530** is configured to cooperate in the anatomical manner with a humeral head.

The articulation surface **530** has a surface finish that is appropriate for an articulation surface that is intended to engage a humeral head. The anchoring surface **520** is generally flat and is intended to contact the cortical bone of a prepared glenoid. The anchoring surface **520** can be coated with a porous tarbecular metallic material such as Wright Medical Technology's ADAPTIS^{™} that can promote bone tissue ingrowth to enhance bonding of the glenoid implant **500** to glenoid after the implant is press fitted into a recess prepared in a glenoid.

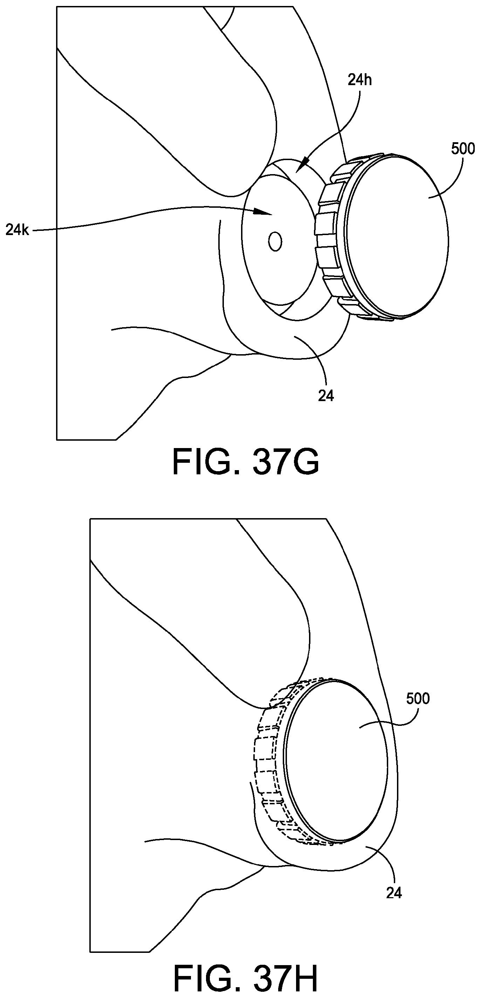

To enable the press fitting feature of the glenoid implant **500,** the annular side wall **511** comprises a plurality of retaining legs **512** that are provided along the annular side wall **511.** glenoid implant **500.** In some embodiments, the retaining legs **512** are extensions of the annular side wall **511** that are folded over towards the articulation surface **530** so that each of the retaining legs **512** are formed as a U-shaped leaf spring as shown in the cross-sectional view in FIG. 37C. Each of the U-shaped leaf spring form of the retaining legs **512** is in an open configuration and is elastically compressible in the radially inward direction. The U-shape is open toward the articulation surface **530** side of the implant **500.** To press fit the implant **500** into a shallow recess **24h** (See FIG. 37G) formed in a glenoid, the recess should have a diameter that is slightly smaller than the outer diameter of the glenoid implant **500** defined by the outer surface of the retaining legs **512.** Thus, when the glenoid implant **500** is inserted into the shallow annular recess formed in a glenoid, the retaining legs **512** are elastically compressed by the side walls of the circular recess as the implant **500** is squeezed in. The outward spring force exerted against the side walls of the annular recess **24h** by the retaining legs **512** securely holds the implant **500** in the recess. The retaining legs **512** are compressed into the bone improving the quality of the anchoring. Because of the U-shaped configuration of the retaining legs **512** that is open toward the surface of the glenoid **24,** the more the implant **500** is pulled outward from the recess, the more the retaining legs **512** will expand radially outward into the surrounding bone and securely hold the implant **500** in the implanted position.

The glenoid implant **500** can be made of a metal such as titanium, CoCr alloy, or a high modulus polymer such as UHMW polyethylene. Preferably, the glenoid implant **500** is integrally formed as a single piece construction. Although not necessary, preferably, the retaining legs **512** and the circular body **510** are formed from a single material. The thickness and diameter of the glenoid implant **500** can be selected to be any desired value for the condition of the surgical site.

In the illustrated example shown in FIG. 37A-37C, the glenoid implant **500** has a generally circular or disk-like outline shape. The glenoid implant **500,** however, is not limited to such circular shape and can be provided to have any desired non-circular outline shape. For example, the glenoid implant **500** can have a glenoid-like outline shape similar to the implant **300** shown in FIG. 18 and implant **400** shown in FIG. 34A.

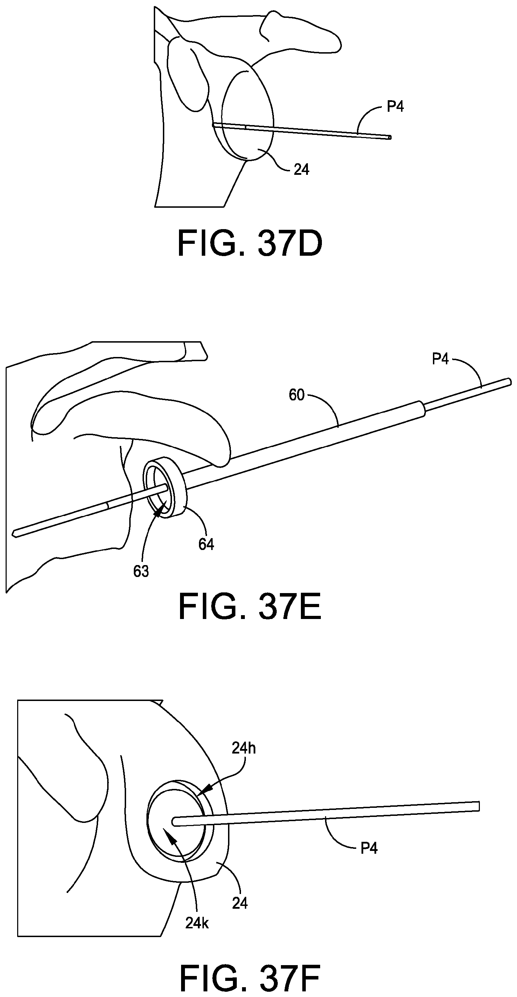

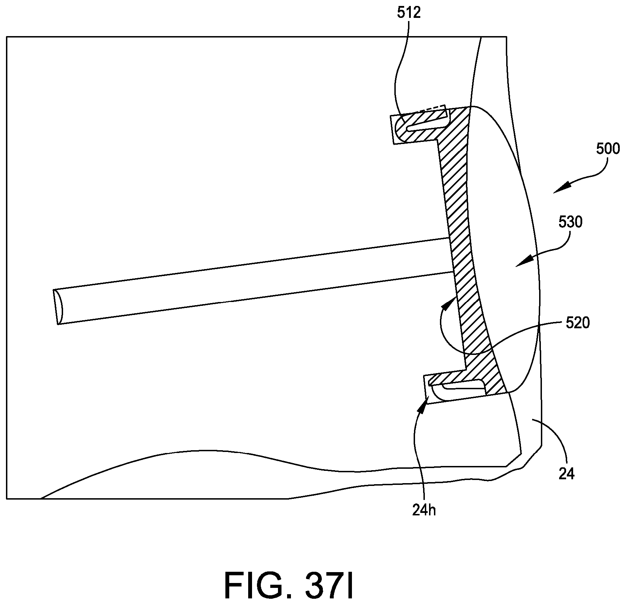

FIGS. 37D-37F are illustrations showing an example of a procedure for preparing a glenoid to receive the glenoid implant of FIGS. 37A-37B. Referring to FIG. 37D, a pin **P4** is placed at a desired location in a glenoid **24.** An appropriate pin guide instrument or Wright Medical Technology's Blueprint^{™} 3D surgical planning system can be used to place the pin **P4** at the desired location. Referring to FIG. 37E, Next, a cannulated 2-in-1 reamer **60** is slid over the pin P4 to form a circular recess in the glenoid **24** that includes a circular substantially flat surface **24k** surrounded by an annular recess **24h** along the perimeter as shown in detail in FIG. 37F. The circular flat surface **24k** is formed to accommodate the anchoring surface **520** of the implant **500** when the implant is press-fitted into the recess. The annular recess **24h** receives the retaining legs **512** of the implant **500.** The reamer **60** comprises an annular cutting ring **64** with a disc shaped body **63** having a circular flat surface on the bone-facing side. The circular flat surface on the bone-facing side of the cutting ring 64 is also an abrasive surface that cuts the glenoid. Referring to FIG. 37G, after the circular recess is formed, the pin **P4** is removed and the glenoid implant **500** is press-fit into the recess. FIG. 37H shows the fully seated implant **500.** FIG. 37I is a cross-sectional view of the fully seated glenoid implant 500.

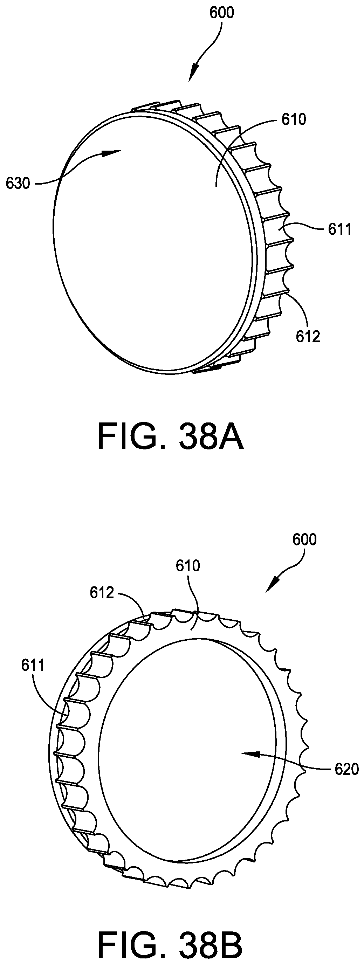

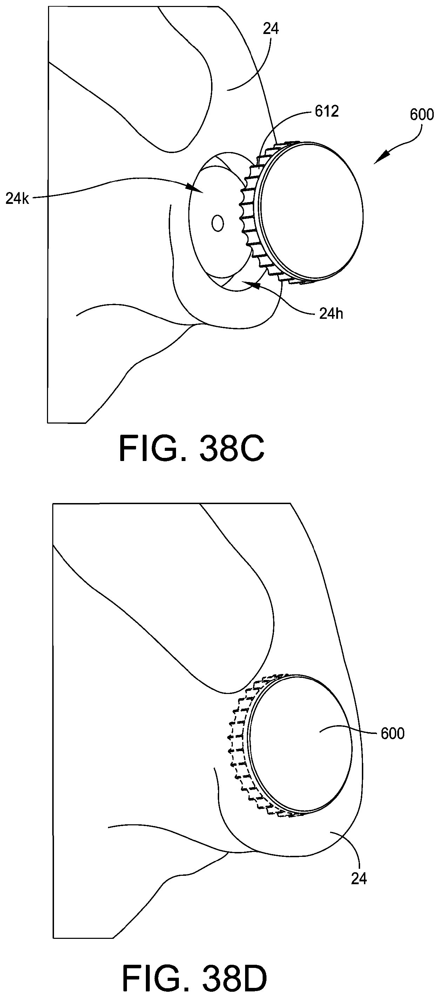

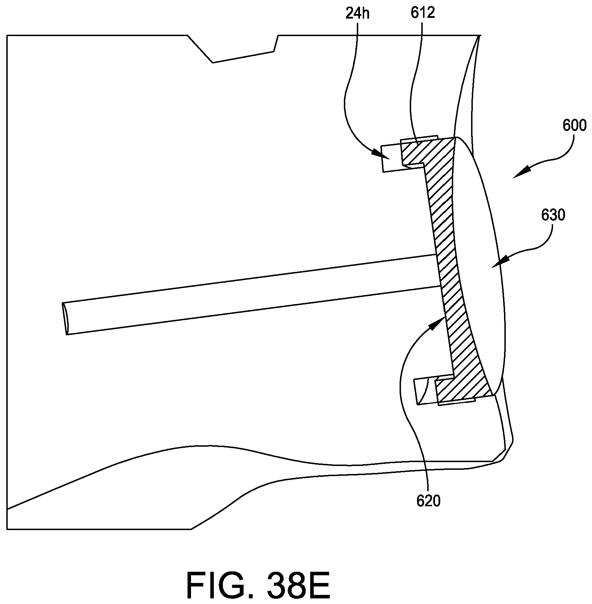

Referring to FIG. 38A-38B, a press-fit glenoid implant **600** according to another embodiment is provided. The glenoid implant **600** is configured to be securely press fitted into a recess prepared into a glenoid to replace damaged natural articulation surface. The glenoid implant **600** comprises a circular body **610** comprising an articulation surface **630** on one side, a base surface **620** on the opposite side, and an annular side wall **611** extending around the periphery of the circular body **610.** The annular side wall **611** is a fluted surface that comprises a plurality of grooves with blades **612** formed in between two adjacent grooves. The glenoid implant **600** is intended to be press-fit into a circular recess prepared in the glenoid. **24.** Similar to the circular recess shown in FIGS. 37F-37G for the glenoid implant **500,** the circular recess prepared for the glenoid implant **600** also comprises a circular flat surface **24k** surrounded by an annular recess **24h** to accommodate the contour of the base surface **620** of the press-fit glenoid implant **600.** This can be seen in the cross-sectional view in FIG. 38E of the press-fit glenoid implant **600** fully seated in the circular recess. The plurality of blades **612** interfere with the surrounding bone to accomplish a secure press-fitting.

The articulation surface **630** is contoured with a generally concave surface that emulates the natural articulation surface of a glenoid. The articulation surface **630** is configured to cooperate in the anatomical manner with the humeral head whether it be a natural one or a prosthetic one.

The articulation surface **630** has a surface finish that is appropriate for an articulation surface that is intended to engage a natural humeral head or a prosthetic humeral head. The anchoring surface **620** is generally flat and is intended to contact the cortical bone of a prepared glenoid. The anchoring surface **620** can be coated with a porous trabecular metallic material such as Wright Medical Technology's ADAPTIS^{™} that can promote bone tissue ingrowth to enhance bonding of the glenoid implant **600** to glenoid after the implant is press fitted into a recess prepared in a glenoid.

The procedure for preparing a glenoid **24** for implanting the press-fit implant **600** into the glenoid **24** is similar to the procedure illustrated in FIGS. 37D-F discussed above. The procedure is used to form a circular recess that comprises the circular flat surface **24k** surrounded by the annular recess **24h.** Then, the implant **600** is press-fit into the circular recess. FIG. 38D and the cross-section in FIG. 38E show illustrations of a fully seated glenoid implant **600.**

The glenoid implant **600** can be made of a metal such as titanium, CoCr alloy or PEEK. Preferably, the glenoid implant **600** is integrally formed as a single piece construction. The thickness and diameter of the glenoid implant **600** can be selected to be any desired value for the condition of the surgical site.

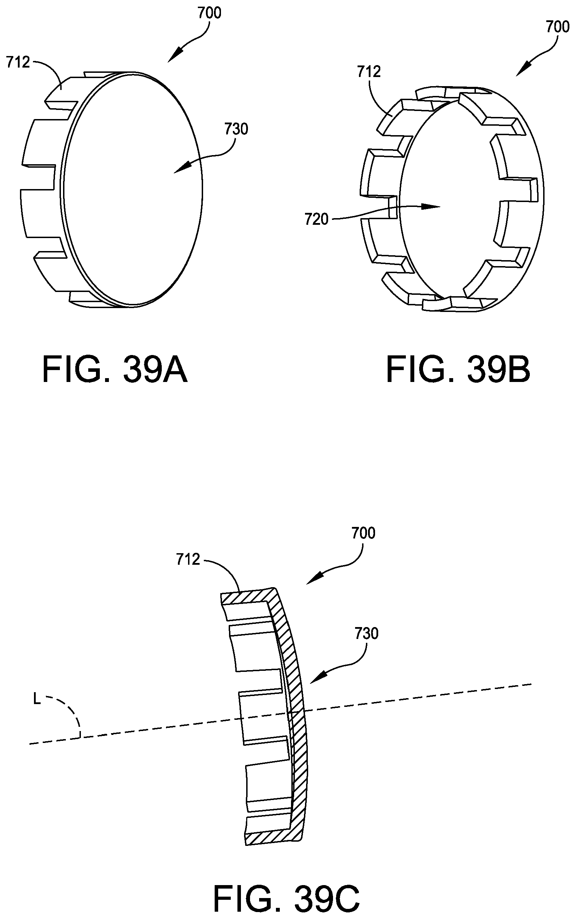

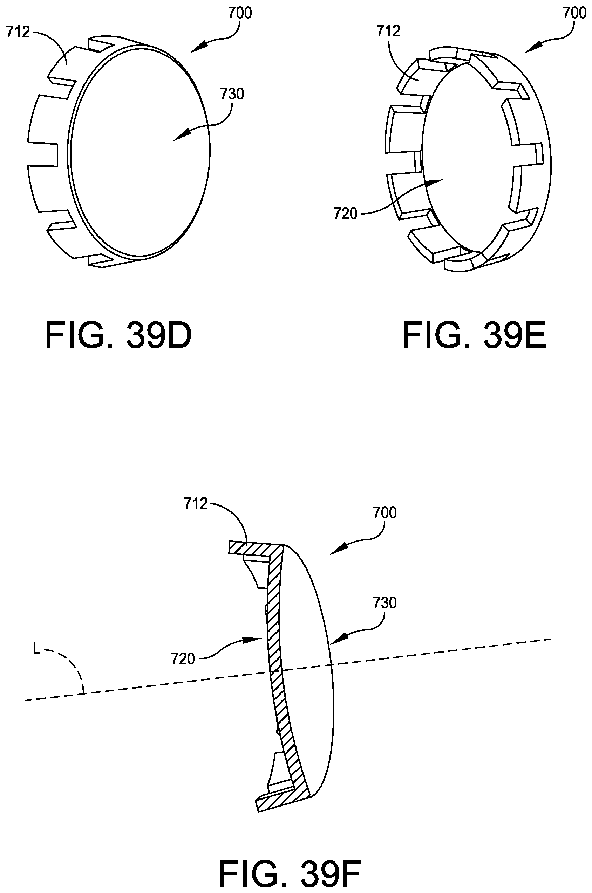

FIGS. 39A-39F are illustrations showing a glenoid implant **700** according to another embodiment of the present disclosure. The glenoid implant **700** comprises a circular body **710** comprising an articulation surface **730** on one side, an anchoring surface **720** on the opposite side, and a plurality of flexible legs **712** extending from the periphery of the circular body **710** toward the direction away from the articulation surface **730.** Thus, the overall shape of the glenoid implant **700** generally resembles a bottle cap.

The glenoid implant **700** can be transformed from its pre-implant configuration to its implanted configuration by plastic deformation. FIGS. 39A-39C are illustrations showing the glenoid implant **700** in its pre-implant configuration and FIGS. 39D-39F are illustrations showing the glenoid implant **700** in its implanted configuration.

In the pre-implant configuration, the circular body **710** is in a shallow dome-like configuration so that the articulation surface **730** is convex and the anchoring surface **720** on the opposite side is concave as shown in FIGS. 39A-39C. Preferably, the flexible legs **712** extend from the periphery of the circular body **710** while being substantially parallel to or towards the longitudinal axis **L** of the implant **700.** The longitudinal axis **L** is defined through the center of the circular body **710.** Being substantially parallel here means at parallel or almost parallel. This configuration can be seen in the cross-sectional view in FIG. 39C. Maintaining the flexible legs **712** substantially parallel to the longitudinal axis **L** allows the implant **700** to be inserted into the glenoid **24** prepared with a recess.

In the implanted configuration, the circular body **710** of the implant has been plastically deformed so that it is now curved in opposite direction from its pre-implant configuration. Now the articulation surface **730** is concave and the anchoring surface **720** on the opposite side is now convex as shown in FIGS. 39D-39F. In this implanted configuration, because of the curved direction of the circular body **710,** the flexible legs **712** around the periphery are now radially extending outward (i.e., away from the longitudinal axis **L**). This configuration helps secure the glenoid implant **700** inside the recess prepared in the glenoid **24** as will be discussed further below.

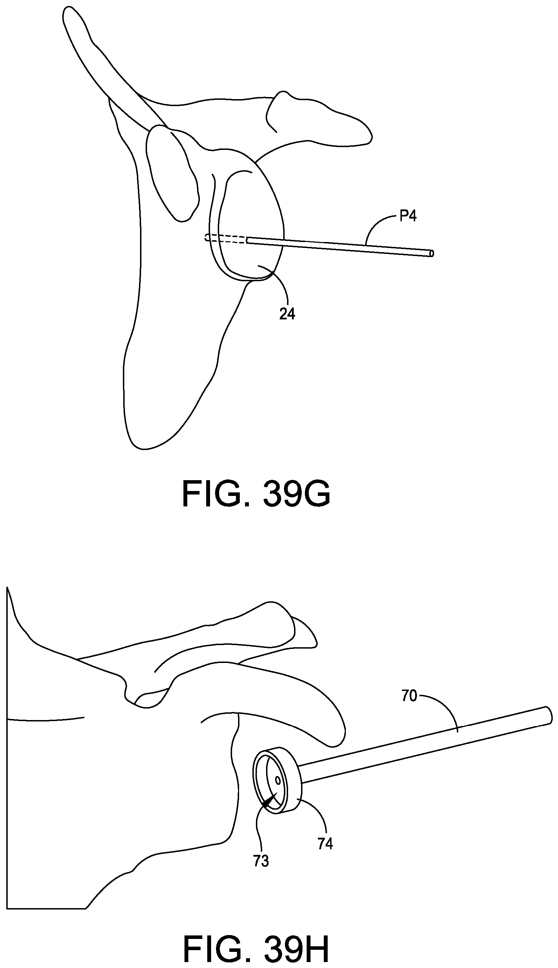

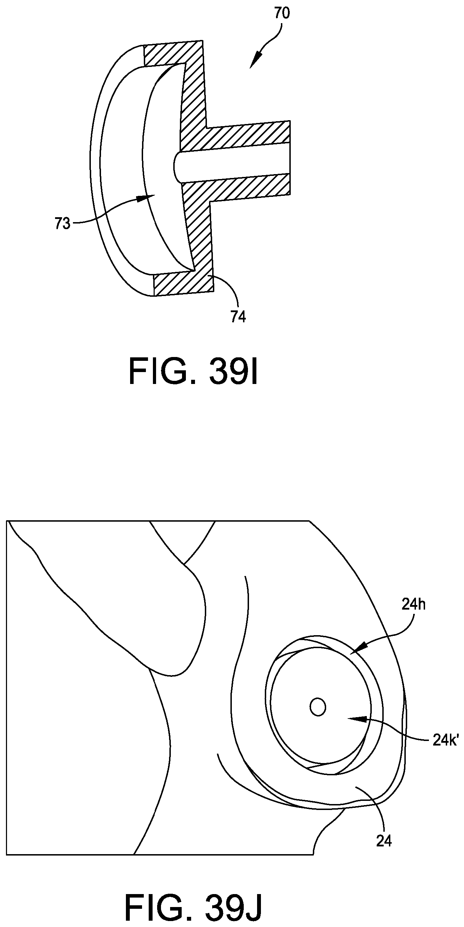

Referring to the illustrations of FIGS. 39G-39L, a procedure for preparing a glenoid **24** and implanting the glenoid implant **700** is now described. First, a recess is formed in the glenoid **24** to receive the glenoid implant **700** using a procedure illustrated in FIGS. 39G FIGS. 37D-37F. Referring to FIG. 39G, a pin **P4** is placed at a desired location in the glenoid **24.** An appropriate pin guide instrument or Wright Medical Technology's Blueprint^{™} 3D surgical planning system can be used to place the pin **P4** at the desired location based on where the glenoid implant **700** should be centered. Referring to FIG. 39H, next, a cannulated 2-in-1 reamer **70** is slid over the pin **P4** to create a circular recess for receiving the glenoid implant **700.** The reamer **70** comprises an annular cutting ring **74** with a disc shaped body having a circular abrading surface **73** on the bone-facing side. The circular abrading surface **73** is not flat but has a curvature as shown in the detailed cross-sectional view in FIG. 39I. This curved surface **73** is convex toward the bone surface it is grinding. Therefore, the 2-in-1 reamer **70** forms a recess in the glenoid **24** that includes a circular concave surface **24k** surrounded by an annular recess **24h** along the perimeter as shown in detail in FIG. 39J. The circular concave surface **24k** is formed to accommodate the anchoring surface **720** of the implant **700** when it is in the implanted configuration.

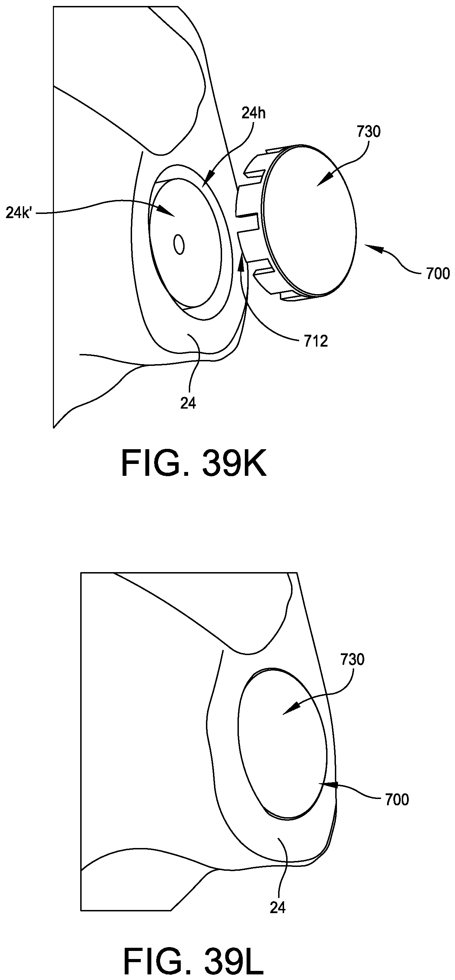

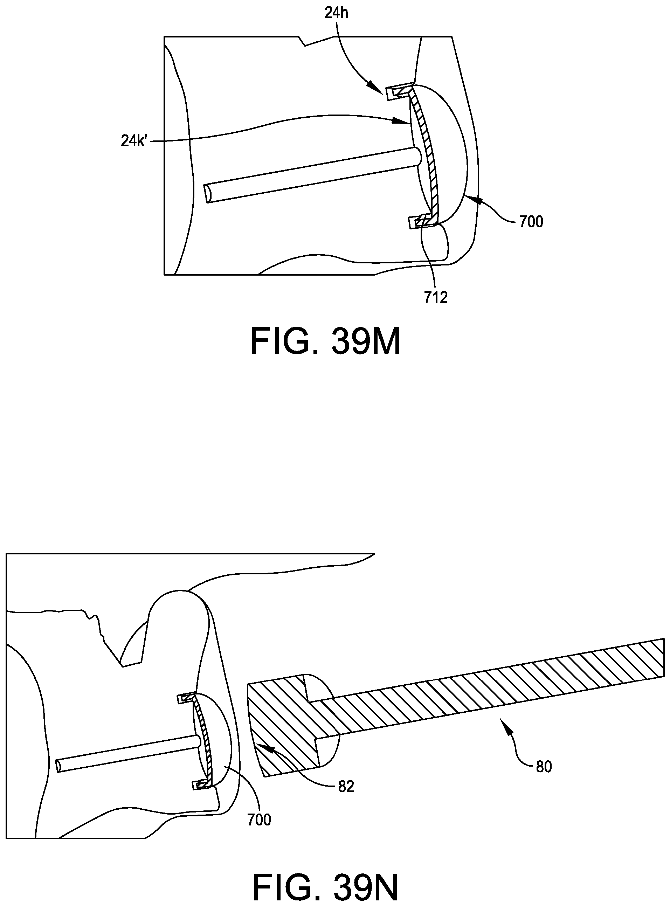

Referring to FIGS. 39K and 39L, after the recess of appropriate size is formed in the glenoid **24,** the glenoid implant **700** in the pre-implant configuration is inserted into the recess **24h.** The flexible legs **712** of the implant **700** are received into the annular recess **24h.** As shown, the articulation surface **730** is convex. Because the flexible legs **712** extend substantially parallel to or towards the longitudinal axis **L** of the implant **700,** they do not interfere when inserted into the recess **24h.** FIG. 39M shows a cross-sectional view of the glenoid implant **700** after it is inserted into the recess.

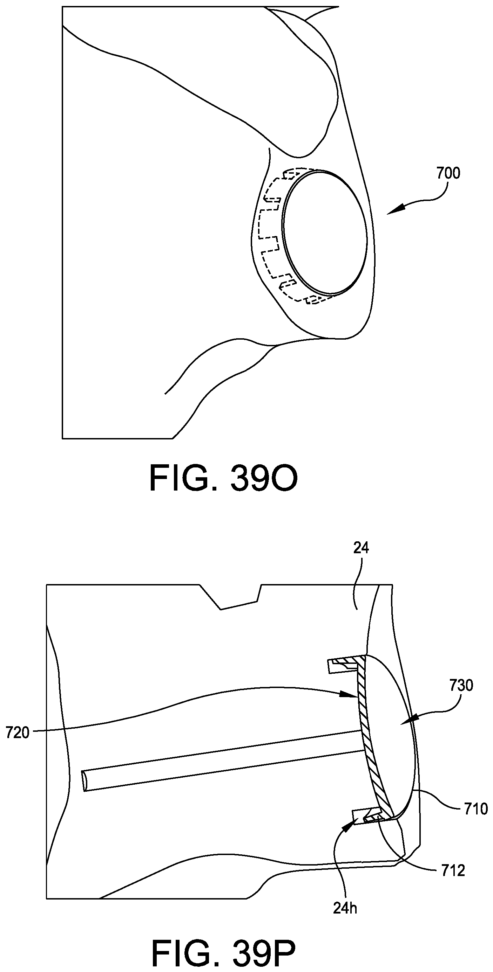

Referring to FIG. 39N, next, an impactor **80** is used to press down onto the articulation surface **730** to plastically deform the implant **700** into its implanted configuration. The impactor **80** comprises a convex tip **82** that has a curvature that substantially matches the convex curvature of the articulation surface **730** after the implant **700** is deformed into its implanted configuration. FIGS. 39O and 39P show the implant **700** after it has been deformed into its implanted configuration. The implant body **710** is now deformed into the implanted configuration so that the articulation surface **730** is concave. The anchoring surface **720** on the opposite side of the implant **700** is now convex and in contact with the concave surface of the circular concave surface **24k** of the recess in the glenoid **24.**

In the implanted configuration of the glenoid implant **700,** the articulation surface **730** is contoured with a generally concave surface that emulates the natural articulation surface of a glenoid. The articulation surface **730** is configured to cooperate in the anatomical manner with the humeral head whether it be a natural one or a prosthetic one.

The articulation surface **730** has a surface finish that is appropriate for an articulation surface that is intended to engage a natural humeral head or a prosthetic humeral head. The anchoring surface **720** is generally flat and is intended to contact the cortical bone of a prepared glenoid. The anchoring surface **720** and/or the flexible legs **712** can be coated with a porous trabecular metallic material such as Wright Medical Technology's ADAPTIS^{™} that can promote bone tissue ingrowth to enhance bonding of the glenoid implant **700** to glenoid after the implant is press fitted implanted.

The implant **700** can be made of a metal such as CoCr, Nitinol, or titanium. Preferably, the glenoid implant **700** is integrally formed as a single piece construction. Although not necessary, preferably, the retaining legs **712** and the circular body **710** are formed from a single material. The thickness and diameter of the glenoid implant **700** can be selected to be any desired value for the condition of the surgical site. Because of the high modulus of the material forming the implant **700,** when the implant is being deformed into the implanted configuration from the pre-implant configuration, the transition happens in a snap as the force exerted by the impactor **80** on the convex anchoring surface **730** reaches a threshold level and the implant's body **710** pops from the pre-implant configuration to the implanted configuration.

As mentioned previously, when the implant **700** is in its implanted configuration shown in FIGS. 39D-39F, the flexible legs **712** radially flare outward. Thus, as shown in the cross-sectional view in FIG. 39P, when the implant **700** is transformed into its implanted configuration while seated within the recess in the glenoid **24,** the legs **712** are butted against the outer wall of the annular recess **24h** as they flare outward. This secures the implant within the recess. The porous trabecular metallic material such as Wright Medical Technology's ADAPTIS^{™} that can be coated on the legs **712** and/or the anchoring surface **720** further enhance the long term securement of the implant **700** in the glenoid **24.**

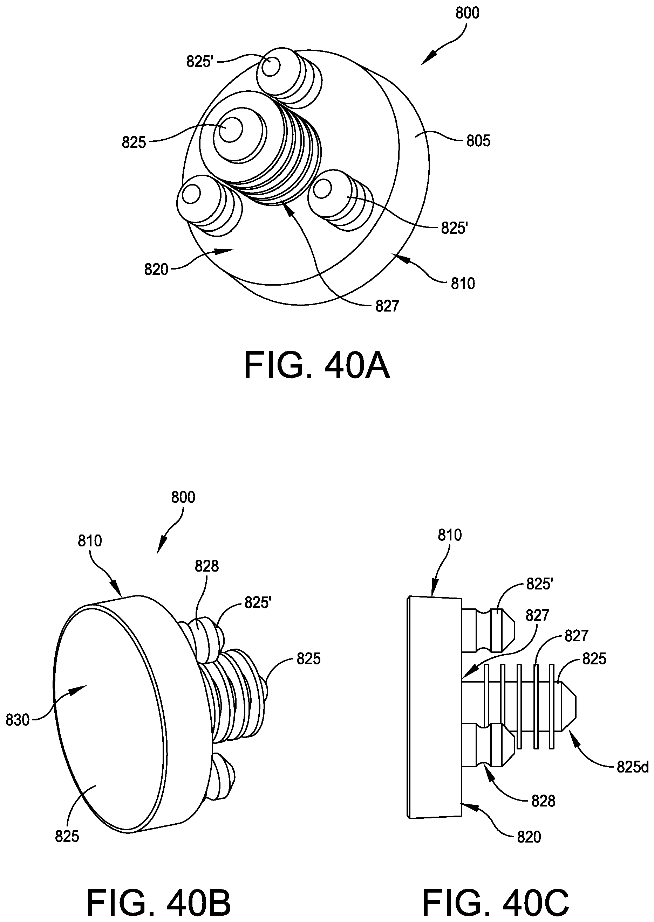

Referring to FIGS. 40A-41F, embodiments of inlay glenoid implants configured to provide improved fixation to glenoid that are integrally formed of a high modulus polymer material, such as UHMWPE or PEEK, are disclosed. These implants will be referred to herein as "all-polymer" implants. The all-polymer glenoid implants can comprise one or more peripheral fixation feature that is provided along the periphery (or circumference) of the implant body or even extend beyond the outer periphery of the implant body. Such placement of the fixation features improves the implants' stability, especially lateral stability, and the quality of fixation.

In some embodiments, the one or more peripheral fixation features can be tapered outer side wall profile, that enable interference fitting along the periphery of the implant body. In other embodiments, the one or more peripheral fixation features can be one or more anchoring elements that extend from the bottom or base surface of the implant body, such as posts, pegs, finned anchors, etc., that are positioned at locations beyond the outer periphery of the implant body. These fixation features can engage the glenoid with mechanical interference fitting, partial interference fitting, no interference fitting, or any combination thereof. These fixation features can also be augmented with bone cement. The fixation features augmented with bone cement can be provided with or without cement pockets.

Additionally, the all-polymer glenoid implants can further comprise conventional anchoring elements that extend from the base surface of the implant body, such as, posts, pegs, finned anchors, keels, etc., in addition to the peripheral fixation features.

The outer profile of the all-polymer glenoid implant can be tapered (i.e. frustoconical), straight without a taper (i.e. perpendicular to the base surface (the face opposite from the articulation surface) where the base surface is flat), or have a lip that creates an interference with the bone. The bone cavity prepared for receiving the glenoid implant can have an undercut created by an instrument that will mate with the interfering lip.

According to some embodiments, the all-polymer glenoid implant can also comprise one or more through holes so that bone screw(s) can be used for additional fixation, if appropriate.

According to some embodiments, the base surface of the all-polymer glenoid implant can be flat, concave, or convex.
Shown in FIGS. 40A-40C is an embodiment of an all-polymer glenoid implant **800** that is configured to be implanted into a glenoid in an inlay configuration. The glenoid implant **800** comprises a substantially circular disk-like body **805** having an articulation surface **830,** an anchoring base surface **820** on the opposite side, and a side wall **810** extending between the two. The glenoid implant **800** can be configured to have a shape that maximizes the articulation surface of the glenoid **24** that is replaced by the glenoid implant **800.** The articulation surface **830** is contoured to replicate the natural articulation surface of the glenoid **24.**

For its peripheral fixation feature, the side wall **810** preferably has a tapered profile, that enables interference fitting along the periphery of the implant body when the implant **800** is implanted into a recess prepared in the glenoid **24.** The taper of the side wall **810** is such that the articulation surface **830** is larger in diameter than the anchoring base surface **820.** Thus, the body **805** has a shallow frusto-conical shape. The interference fitting provided by the tapered side wall **810** is a fixation feature that extends radially outward beyond the periphery of the substantially circular shaped body **805.**

The glenoid implant **800** comprises additional features that helps secure the implant **800** in the glenoid **24.** For example, extending from the anchor surface **820** is at least one finned anchor **825** and a plurality of stabilizing posts **825'.** The articulation surface **830** is generally concave and is configured to engage a humeral head.

The finned anchor **825** and the stabilizing posts **825'** of the glenoid implant **800** extend from the base surface **820** and secure the glenoid component **800** to the glenoid **24.** The stabilizing posts **825'** are positioned radially outward from the finned anchor **825** so that the stabilizing posts **825'** are located along the peripheral region of the glenoid implant **800** and offer stability to the glenoid implant **800** after implantation. In the illustrated example embodiment, the finned anchor **825** and the stabilizing posts **825'** extend substantially perpendicularly from the base surface **820.** In some embodiments, the finned anchor **825** can extend at various other angles relative to the base surface **820.** The finned anchor **825** is preferably positioned substantially at the center of the base surface **820** and is in the form of a cylindrical shaft having a proximal end **825p** and a distal end **825d.** (See FIG. 40C). The finned anchor **825** is attached to the base surface **820** at the proximal end **825p** and tapers at the distal end **825d** to facilitate insertion of the finned anchor **825** into an anchor receiving hole prepared in the glenoid **24.** In one embodiment, the distal end **825d** of the finned anchor **825** includes a conical tip or other shape that facilitates insertion into glenoid **24,** with or without a pre-drilled hole. In the example shown in FIGS. 40A-40C, the distal end **825d** has a conical tip.

In the illustrated example, the finned anchor **825** comprises a substantially constant diameter and further comprises a plurality of fins **827** that extend radially outward. The each of the fins **827** are spaced apart from each other along the length of the finned anchor **825.** The fins **827** can be equally spaced or the spacing can be varied if desired.

The fins **827** are flexible and are configured to bend or deform when force is exerted against them. Deformation of the fins **827** can be plastic or elastic. In some embodiments, the fins **827** are formulated to deform plastically upon insertion into the glenoid **24** and assume a generally curved configuration once implanted. In some embodiments, the fins **827** are formulated to deform elastically upon insertion into the glenoid **24** and constantly exert some amount of force against the surrounding bone once implanted as the fins try to return to their undeformed configuration.

In some embodiments, the finned anchor **825** and its fins **827** can be integrally formed with the body **805.** For example, the glenoid implant **800** can be molded as a single unitary structure or machined from a monolithic piece of polymer material. In other embodiments, the finned anchor **825** and the body **805** are separate components. In an alternate embodiment, the body **805** can be molded from a first material while the finned anchor **825** and its fins **827** are molded from a second material. In this embodiment, the second material preferably has a higher stiffness than the first material.

The stabilizing posts **825'** prevent the glenoid implant **800** from moving relative to the glenoid **24** once the implant **800** is implanted in the glenoid **24.** The stabilizing posts **825'** preferably extend substantially perpendicular to the base surface **820** of the implant **800.** Each of the stabilizing posts **825'** includes a body having a proximal end **825p'** and a distal end **825d'.** Each of the body of the stabilizing posts **825'** is attached at its proximal end **825p'** to the base surface **820** of the implant body **805.** The stabilizing posts **825'** can also include an indent or a series of indents to accept and lock in bone cement, maintaining the stabilizing posts **825'** in position.

The stabilizing posts **825'** are preferably shorter than the finned anchor **825.** Similar to the distal end **825d** of the finned anchor **825,** the distal ends **825'd** of the stabilizing posts **825'** can also be tapered to facilitate insertion of the stabilizing posts **825'** into holes prepared in the glenoid **24.** In some embodiments, the distal ends **825'd** of the stabilizing posts **825'** have a conical tip, or other shape that facilitates insertion into the glenoid **24,** with or without pre-drilled hole.

The stabilizing posts **825'** can be arranged in any configuration on the base surface **820.** In one embodiment, the stabilizing posts **825'** positioned such that one of the stabilizing posts **825'** is positioned farther from the finned anchor **825** than the other stabilizing posts **825'.** In another embodiment, the stabilizing posts **825'** are positioned around the finned anchor **825** along a periphery of the substantially equidistant from the finned anchor **825** and each adjacent stabilizing posts **825'.**

The structure and function of the finned anchor **825** are similar to those of the similar anchor described in United States patent No. 10,524,922, the disclosure of which is incorporated herein by reference.

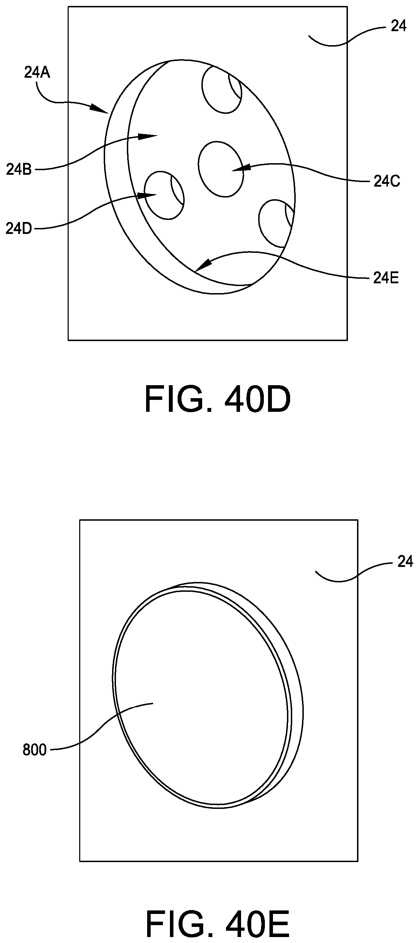

Referring to FIGS. 40D-40E, to receive the glenoid implant **800,** a recess **24A** sized and shaped appropriately for the glenoid implant **800** is reamed into a glenoid **24.** The recess comprises a bottom surface **24B** into which a hole **24C** is drilled to receive the anchor **825** and additional holes **24D** are drilled to receive the stabilizing posts **825'.** The holes 24C and 24D have the appropriate diameter and depth to receive the corresponding finned anchor **825** or the stabilizing post **825'.** The recess **24A** has a sidewall **24E** that is tapered to match the taper of the side wall **810** of the glenoid implant **800.** FIG. 40E shows the glenoid implant **800** seated in the recess **24A.**

In some embodiments, each of the stabilizing posts **825'** can comprise one or more bone cement pockets **828.** When the glenoid implant **800** is being secured into the corresponding recess **24A** prepared in the glenoid **24,** bone cement can be used to enhance the securement of the implant. Each of the pockets **828** can hold an amount of bone cement.

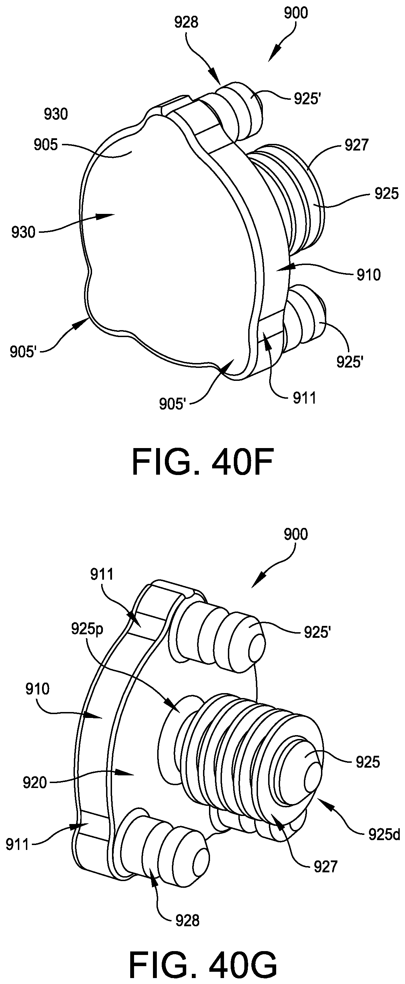

Shown in FIGS. 40F-40G is an all-polymer glenoid implant **900** configured for an inlay implantation into a glenoid according to another embodiment. The glenoid implant **900** comprises a body **905** comprising an articulation surface **930,** a base surface **920** on the opposite side, and a side wall **910** extending between the two. The glenoid implant **900** can be configured to have a shape that maximizes the articulation surface of the glenoid **24** that is replaced by the glenoid implant **900.** The articulation surface **930** is contoured to replicate the natural articulation surface of the glenoid **24.**

The side wall **910** can have a tapered profile, that enables interference fitting along the periphery of the implant body when the implant **900** is implanted into a recess prepared in the glenoid **24.** The taper of the side wall **910** is such that the articulation surface **930** is larger in diameter than the base surface **920.**

In this embodiment, the body **905** has a plurality of protruding portions **905'** that extend radially outward beyond the outer periphery (or circumference) of the substantially circular shape of the body **905** and a fixation feature such as a stabilizing post **925'** is provided on each of the protruding portions **905'** extending from the base surface. Because the protruding portions **905'** allow placement of the fixation features outside the periphery of the body **905,** this configuration enhances the lateral stability of the glenoid implant **900** in the bone and help mitigate rocking of the implant.

The glenoid implant **900** can further comprise one or more additional non-peripheral fixation features that helps secure the implant **900** in the glenoid **24.** The non-peripheral fixation features can comprise any one of a post, a finned anchor, or a keel, etc. For example, in the illustrated example glenoid implant **900,** extending from the anchor surface **920** is a finned anchor **925.** The articulation surface **930** is generally concave and is configured to engage a humeral head.

The finned anchor **925** and the stabilizing posts **925'** of the glenoid implant **900** extend from the base surface **920** and secure the glenoid component **900** to the glenoid **24.** The stabilizing posts **925'** are positioned radially outward from the finned anchor **925** so that the stabilizing posts **925'** are located along the peripheral region of the glenoid implant **900** and offer stability to the glenoid implant **900** after implantation.

In the illustrated example embodiment, the finned anchor **925** and the stabilizing posts **925'** extend substantially perpendicularly from the base surface **920.** In some embodiments, the finned anchor **925** can extend at various other angles relative to the base surface **920.** The finned anchor **925** is preferably positioned substantially at the center of the base surface **920** and is in the form of a cylindrical shaft having a proximal end **925p** and a distal end **925d.** (See FIG. 40G). The finned anchor **925** is attached to the base surface **920** at the proximal end **925p** and tapers at the distal end **925d** to facilitate insertion of the finned anchor **925** into an anchor receiving hole prepared in the glenoid **24.** In one embodiment, the distal end **925d** of the finned anchor **925** includes a conical tip or other shape that facilitates insertion into glenoid **24,** with or without a pre-drilled hole. In the example shown in FIGS. 40F-40G, the distal end **925d** has a conical tip.

In the illustrated example, the finned anchor **925** comprises a substantially constant diameter and further comprises a plurality of fins **927** that extend radially outward. The each of the fins **927** are spaced apart from each other along the length of the finned anchor **925.** The fins **927** can be equally spaced or the spacing can be varied if desired.

The fins **927** are flexible and are configured to bend or deform when force is exerted against them. Deformation of the fins **927** can be plastic or elastic. In some embodiments, the fins **927** are formulated to deform plastically upon insertion into the glenoid **24** and assume a generally curved configuration once implanted. In some embodiments, the fins **927** are formulated to deform elastically upon insertion into the glenoid **24** and constantly exert some amount of force against the surrounding bone once implanted as the fins try to return to their undeformed configuration.

In some embodiments, the finned anchor **925** and its fins **927** can be integrally formed with the body **905.** For example, the glenoid implant **900** can be molded as a single unitary structure or machined from a monolithic piece of polymer material. In other embodiments, the finned anchor **925** and the body **905** are separate components. In an alternate embodiment, the body **905** can be molded from a first material while the finned anchor **925** and its fins **927** are molded from a second material. In this embodiment, the second material preferably has a higher stiffness than the first material.

The functions of the finned anchor **925** and the stabilizing posts **925'** are similar to those of the finned anchor **825** and the stabilizing posts **825'** of the glenoid implant **800** except that the stabilizing posts **925'** are located so that they are positioned beyond the periphery of the substantially circular body **905** to further enhance the lateral stability of the implant **900** when implanted in a glenoid **24.**

In some embodiments, each of the stabilizing posts **925'** can comprise one or more bone cement pockets **928.** When the glenoid implant **900** is being secured into the corresponding recess prepared in the glenoid **24,** bone cement can be used to enhance the securement of the implant. Each of the pockets **928** can hold an amount of bone cement.

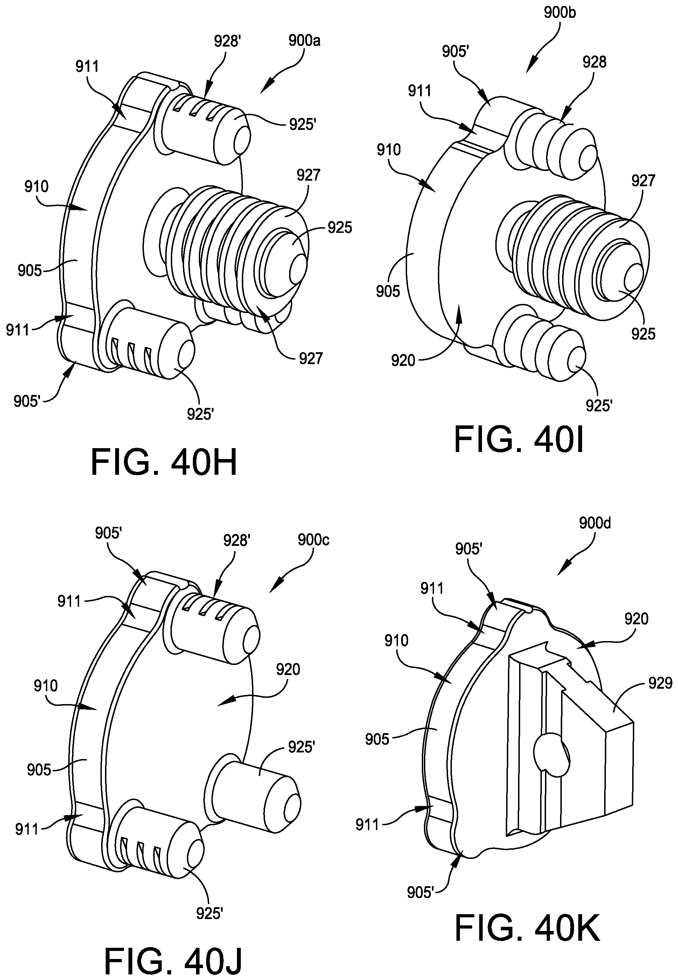

In some embodiments, the glenoid implant **900** can be provided in a variety of configurations to have different types and numbers of fixation elements. For example, the implant **900** can have just one type of fixation elements extending from the anchoring surface **920** (i.e. finned anchor **925,** the stabilizing posts **925',** keel **929,** etc.) as one or more fixation elements provided. In other embodiments, the implant **900** can have different bone cement pockets, or even different types of anchoring elements such as keel. For example, FIG. 40H shows an all-polymer glenoid implant embodiment **900a** in which the stabilizing posts **925'** have one or more bone cement pockets **928'** that are slots cut into the stabilizing posts **925'.** FIG. 40I shows an all-polymer glenoid implant embodiment **900b** that comprises two stabilizing posts **925'** that are located 180° apart from one another. Also, the cement pocket **928** on the stabilizing posts **925'** are grooves. FIG. 40J shows an all-polymer glenoid implant embodiment **900c** that has the same three stabilizing posts **925'** as those in the embodiment of FIG. 40H but does not have any finned anchor **925.** In the all-polymer glenoid implant embodiment **900c,** the base surface **920** can be substantially flat or can have a slightly convex or concave contour. The benefits of a convex contoured base surface **920** can be that such surface is more conforming to natural glenoid articulation, thus requiring less subchondral (good) bone reaming. Convex surface also provides larger contact surface area than a flat base surface. The benefits of a concave contoured base surface **920** is that it may improve the stability of the implant by reversing the "soap-dish" effect that can cause lateral slippage of the implant under peripheral loading conditions. Furthermore, a concave back surface allows the periphery of the implant to be situated deeper in the bone while preserving cortical bone thickness in the center where the forces are the greatest and the primary non-peripheral fixation feature is located.

FIG. 40K shows an all-polymer glenoid implant embodiment **900d** that has a keel **929** rather than finned anchors **925** or stabilization posts **925'.** The keel **929** and its variants are similar to the keel for a glenoid component described in United States patent No. 8,080,063, the contents of which are incorporated herein by reference. In some embodiments, the glenoid implant **900** can be configured with any combination of the fixation features described herein as appropriate.

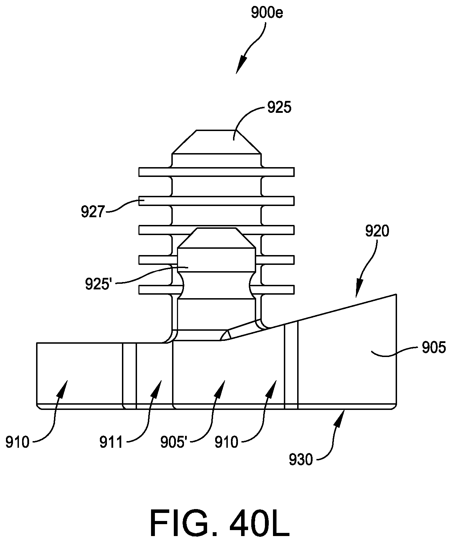

FIG. 40L shows an all-polymer glenoid implant embodiment **900e** where the base surface **920** of the implant can be contoured in any variety of ways to accommodate the condition of the glenoid **24.** In the illustrated example, the base surface **920** is slanted on one side of the implant to form a wedge-shaped profile for the body **905** of the implant **900.**

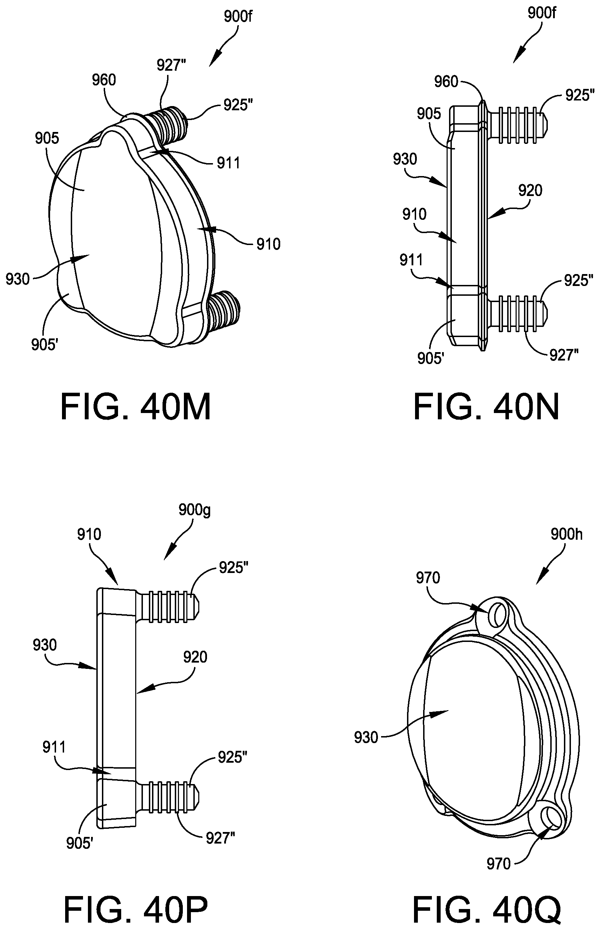

FIGS. 40M and 40N show an all-polymer glenoid implant embodiment **900f** that comprises a plurality of protruding portions **905'** that are provided with mini-finned anchors **925"** extending from the base surface **920** as one or more peripheral fixation features. The glenoid implant **900f** further comprises a protruding lip **960** along the periphery of the base surface **920** side of the body **905** as additional peripheral fixation feature. The protruding lip **960** engages the side **wall 24K** (see the recess 24G in FIG. 40M) of the recess prepared into the glenoid **24** and creates an interference fit to help secure the implant **900f** in the glenoid **24.**

This shape is intended to control the translation of the mating humeral head, allowing it to only translate across the surface in one linear vector. For example, depending on the orientation of the "swept" geometry the glenoid could easily transvers the glenoid surface along an inferior-superior trajectory but meets with resistance to traversing the glenoid in an anterior-posterior motion.

FIG. 40P is a side view of an all-polymer glenoid implant embodiment **900g** that comprises a tapered side wall **910** and a plurality of protruding portions **905'** that are provided with mini-finned anchors **925"** extending from the base surface **920** as the one or more peripheral fixation features. This glenoid implant **900g** does not have any non-peripheral fixation features.

FIG. 40Q shows an all-polymer glenoid implant embodiment **900h** in which a plurality of screw holes **970** are the peripheral fixation feature and does not have any additional non-peripheral fixation features such as posts, finned anchors, or keel. The glenoid implant **900h** is secured to a glenoid using bone screws. In some other embodiments, the glenoid implant can have other fixation features in addition to the screw holes.

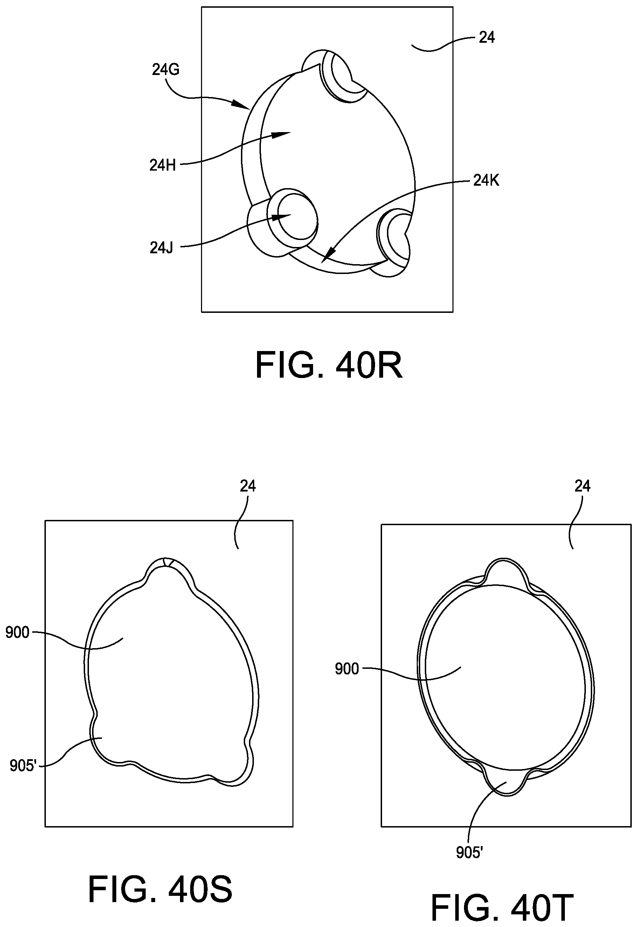

FIG. 40R is an illustration of a glenoid **24** that has been reamed to form a recess **24G** that is sized and shaped appropriately for receiving the glenoid implant **900** example shown in FIG. 40J. The recess **24G** comprises a bottom surface **24H,** side **wall 24K** and holes **24J** that have been drilled along the periphery of the recess **24G** for receiving the stabilizing posts **925'** and to accommodate and engage the protruding portions **905'** that extend radially outward beyond the outer periphery of the substantially circular shape of the implant body **905.**

FIG. 40S is an illustration of the implant **900** of FIG. 40J that has been implanted into the recess **24G.** FIG. 40T is an illustration of the implant **900** of FIG. 40I that has been implanted into a recess, similar to the recess **24G,** that is shaped and sized for the implant **900** of FIG. 40I.

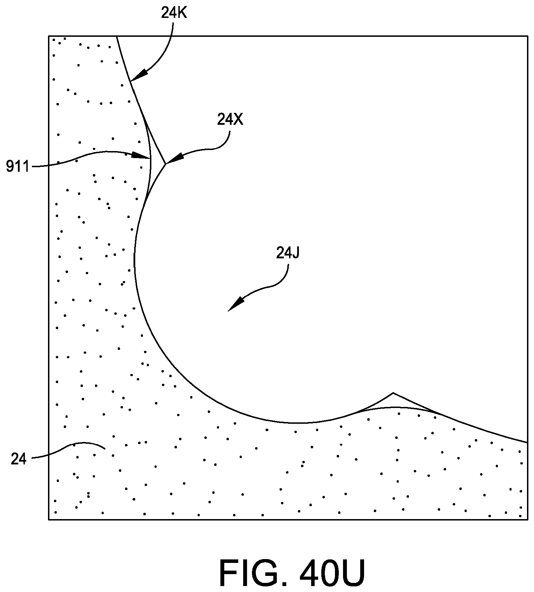

As can be seen in FIGS. 40F-40K, in some embodiments of the glenoid implant **900,** the transition region **911** between the protruding portion **905'** and the remainder of the side wall **910** portion is a curved surface. The outline of the recess **24G** and the holes **24J** formed in the glenoid **24** are shaped to form an interference fitting engagement with the curved transition region **911** of the glenoid implant **900.** This is illustrated in FIG. 40U. FIG. 40U shows the portion of the glenoid **24** around one of the holes **24J** that has been prepared in the glenoid **24.** Where the side **wall 24K** of the recess **24G** meets the hole **24J** is a cusp **24X.** This cusp **24X** and the curved transition region **911** of the glenoid implant **900** overlap each other and thus form an interference fit. In FIG. 40U, the outline of the transition region **911** of the glenoid implant **900** is shown overlapping with the cusp **24X** which enables the interference fit.

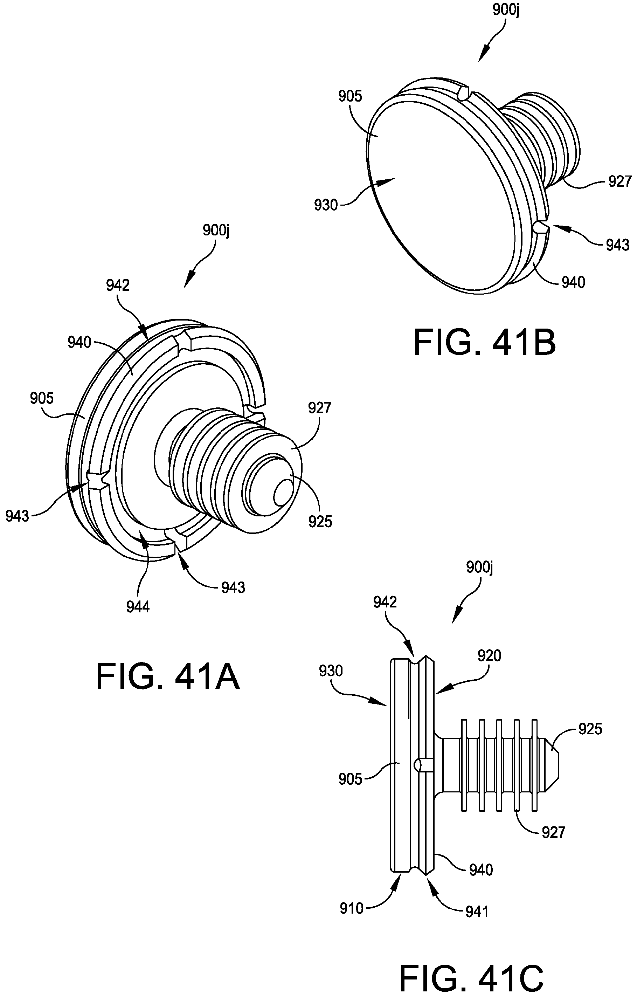

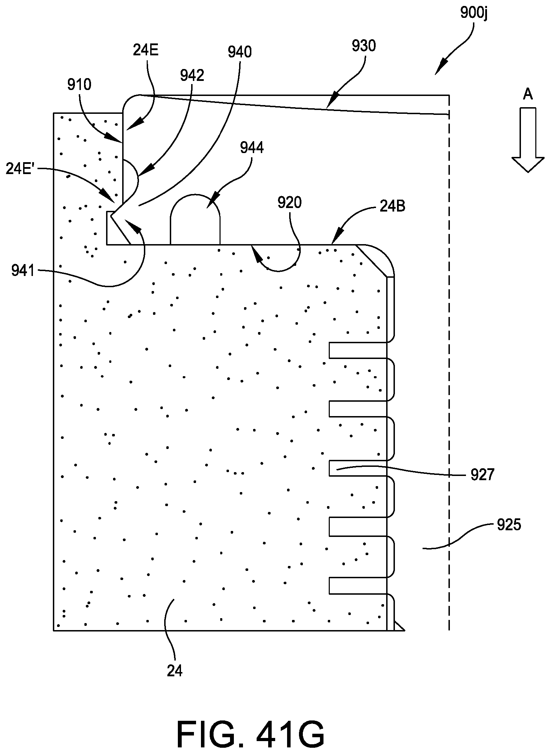

FIGS. 41A-41C show another embodiment of all-polymer glenoid implant **900j.** The glenoid implant **900j** comprises a locking rim structure **940** as its peripheral fixation feature that secures the implant **900j** in a recess prepared in a glenoid **24** by pushing the body **905** of the implant into the recess. The glenoid implant **900j** further comprises a finned anchor **925** as the one or more additional fixation feature. As can be better seen in the side view FIG. 41C, the locking rim **940** is defined by an annular groove **942** formed into the outer side wall **910** of the substantially circular body **905** and an undercut groove **944** formed into the base surface **920** of the body **905.** The locking rim **940** has a larger diameter than the substantially circular body **905** and comprises an edge **941** that protrudes radially outward from the body **905.** The engagement between the locking rim **940** and the recess prepared in a glenoid **24** can be seen in detail in FIG. 41G. FIG. 41G is a partial cross-sectional view of the all-polymer glenoid implant **900j** that is implanted into a recess prepared in a glenoid **24.** The cross-section shows the structure of the locking rim **940.** The annular groove **942** and the undercut groove **944** define the locking rim **940.** The edge **941** of the locking rim **940** protrudes radially outward beyond the side wall **910.** The recess reamed into the glenoid is similar to the recess **24A** shown in FIG 40D and has a side wall **24E** and a bottom surface **24B.** To receive the glenoid implant **900j,** the bottom portion of the side **wall 24E** of the recess is reamed further radially outward with an undercut thus forming an overhang **24E'.** Because the edge **941** of the locking rim **940** protrudes radially outward, the rim **940** has a larger diameter than the diameter of the recess formed by the side **wall 24E.** Thus, as the glenoid implant **900j** is being pushed into the recess in the glenoid **24** in the direction noted by the arrow **A,** the locking rim **940** elastically bends radially inward until the edge **941** clears the overhang **24E'.** Once the edge **941** clears the overhang **24E',** the locking rim **940** snaps back outward into the configuration shown in FIG. 41G where the edge **941** and the overhang **24E'** creates a mechanical lock that secures the glenoid implant **900j** in place. As the glenoid implant **900j** is an all-polymer implant, the polymer material allows the locking rim **940** to elastically bend as described. The modulus of elasticity of the locking rim **940** can be tuned by selecting appropriate polymer formulation.

Alternatively, in some embodiments, the side wall **24E** of the recess in the glenoid **24** can be simply straight without any undercut and the edge 941 of the locking rim **940** simply creates an interference fit with the reamed recess.

According to some embodiments, the locking rim **940** can be further configured with a plurality of compression relief cutouts **943** as shown. The compression relief cutouts **943** divides the locking rim **940** into multiple segments as shown. Preferably, the compression relief cutouts **943** are located at positions that radially symmetric so that the locking rim **940** is divided into equal-sized segments thereby providing radially symmetric compression relief as the glenoid implant **900f** is pushed into the recess in the glenoid **24.**

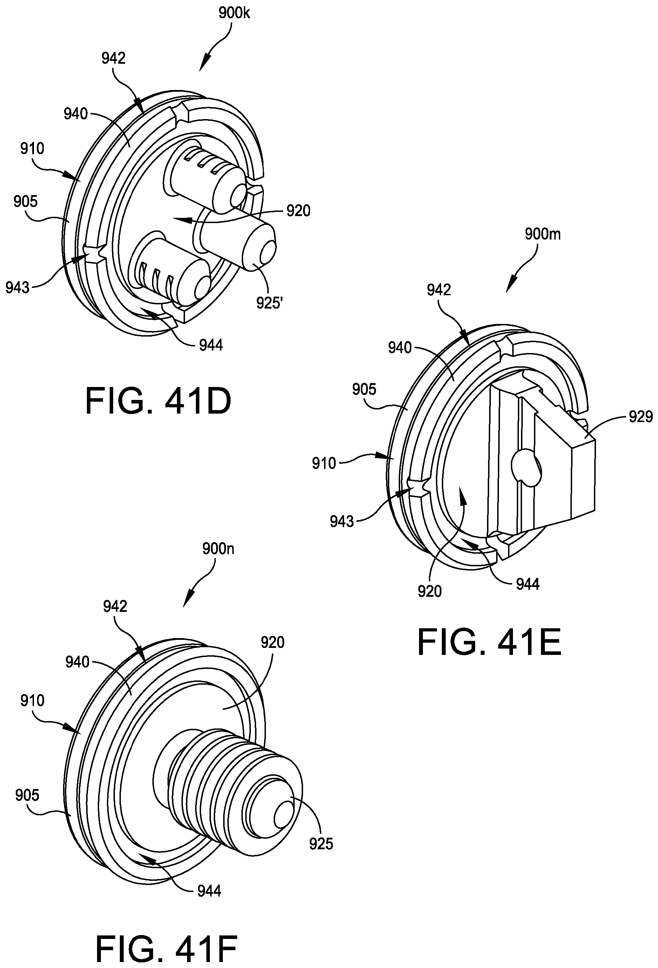

FIGS. 41D-41F show examples of additional embodiments of the all-polymer glenoid implant comprising the locking rim **940** feature as the peripheral fixation feature but are configured with one of a variety of other possible fixation features that extend from the base surface **920.** FIG. 41D shows an all-polymer glenoid implant embodiment **900k** that comprises the locking rim **940** like the glenoid implant **900j** but has three stabilizing posts **925'** extending from the base surface **920** as the one or more additional fixation features. FIG. 41E shows an all-polymer glenoid implant embodiment **900m** that also comprises the locking rim **940** like the glenoid implant **900j** but has a keel **929** extending from the base surface **920** as the one or more additional fixation features. FIG 41F shows an all-polymer glenoid implant embodiment **900n** that also comprises the locking rim **940** like the glenoid implant **900j** but the locking rim **940** does not have the compression relief cutouts.

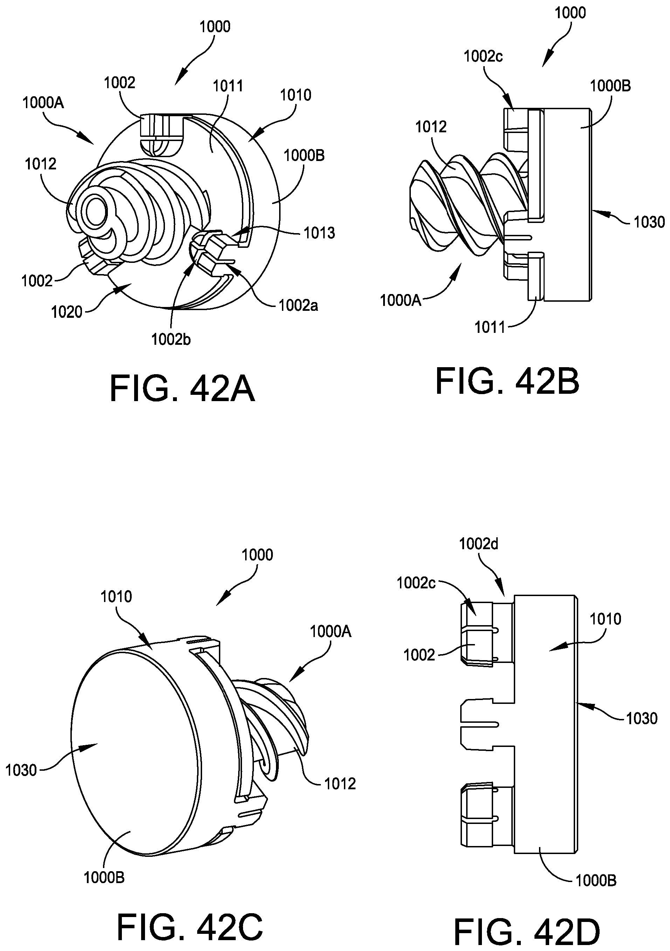

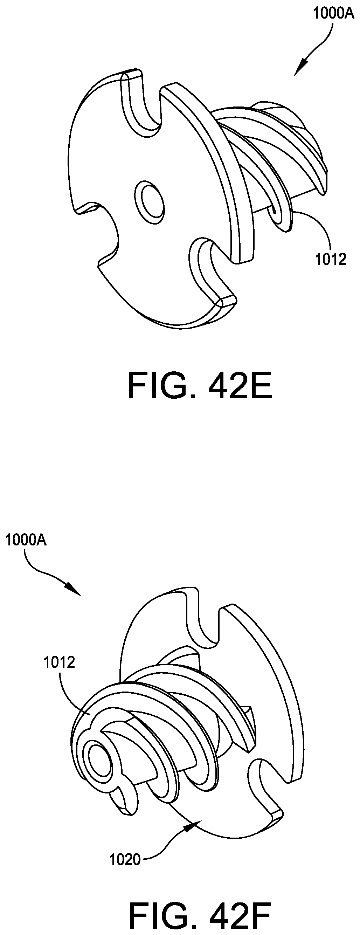

FIGS. 42A-42D show a metal-backed glenoid implant embodiment **1000.** The metal-backed glenoid implant comprises two-piece construction: a metal anchor **1000A,** and a polymer insert **1000B** that are configured to lock into each other during the implantation process. The polymer insert **1000B** can be made of high modulus polymer material, such as UHMWPE or PEEK. The metal anchor **1000A** comprises a circular plate portion **1011** comprising two faces and a threaded screw portion **1012** extending from the center of one of the two faces that is the bone-facing base surface **1020.** The face that is opposite of the base surface **1020** is the one that receives the polymer insert **1000B.** The plate portion **1011** is configured with a plurality of cutouts or notches **1013** along the periphery of the plate portion **1011.** The polymer insert **1000B** comprises an articulation surface **1030** and further comprises a plurality of tabs **1002** extending from the polymer insert **1000B** on the side opposite from the articulation surface **1030.** The tabs **1002** are configured to snap into the corresponding cutouts **1013.** The number and location of the tabs **1002** match the number and location of the plurality of cutouts **1013** on the plate portion **1011.** The polymer insert **1000B** and the metal anchor **1000A** lock into each other by aligning and inserting the tabs **1002** through the cutouts **1013.** FIGS. 42E-42F are illustrations of the metal anchor **1000A** without the polymer insert **1000B.**

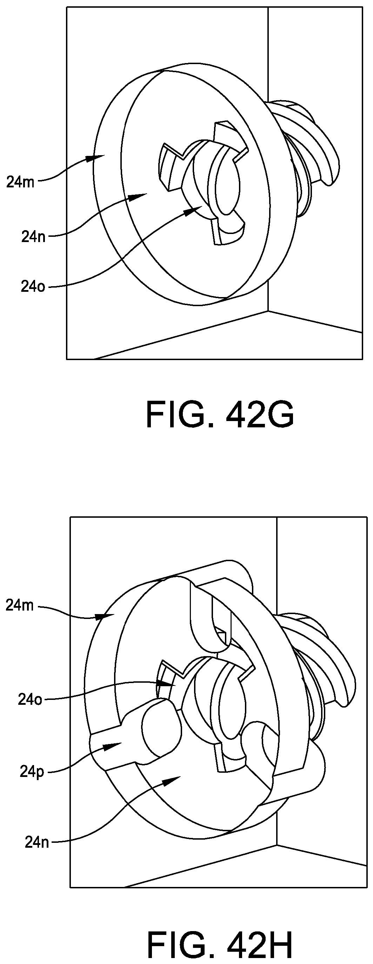

During the implantation process, the metal anchor **1000A** is first screwed into a bone **24** that is prepared with a recess **24m** (See FIG. 42H) that has a tapped threaded hole **24n.** Once the metal anchor **1000A** is threaded in place in the recess 24m, the polymer insert **1000B** is snapped onto the plate portion **1011** of the metal anchor **1000A** by first aligning the plurality of tabs with the cutouts **1013** and pushing the polymer insert **1000B** toward the plate portion **1011** until the tabs **1002** are fully inserted through the cutouts **1013** and lock. Each of the tabs **1002** comprise one or more compression relieving slots **1002a, 1002b** and the leading end **1002c** of the tabs **1002** are larger than the cutouts **1013.** This allows the tabs **1002** to compress as they get squeezed into the cutouts **1013** then spring back to their resting configuration once the polymer insert **1000B** is fully engaged with the metal anchor **1000A.** FIG. 42D is a side view of the polymer insert **1000B.** Each of the tabs **1002** has a leading end **1002c** that is somewhat larger than the opening provided by the cutouts **1013** and a neck portion **1002d** that is sized to match the size of the opening provided by the cutouts **1013.** Thus, when the tabs **1002** are inserted into the cutouts **1013** the compression relieving slots **1002a, 1002b** allow the leading end **1002c** on each of the tabs **1002** to compress ad allow the tabs **1002** to fit through the cutouts **1013.** Once the cutouts **1013** get past the leading end **1002c** and engage the neck portion **1002d,** the leading end **1002c** decompresses back to its resting state creating an interference fit between the leading end **1002c** and the plate portion **1011** and hold the polymer insert **1000B** and the metal anchor **1000A** together. As can be seen in FIGS. 42A-42C, when the metal anchor **1000A** and the polymer insert **1000B** are assembled together, the leading ends **1002c** of the tabs **1002** protrude from the base surface **1020** of the metal anchor **1000A.**

FIGS. 42G-42H are illustrations showing an example of a circular recess **24m** that would be prepared into a bone for receiving the metal-backed glenoid implant **1000.** FIG. 42G-42H show a graphical rendering of the 3-dimensional form of just the surface of the bone after the recess **24m** is formed and the bulk bone material is removed. Referring back to FIG. 42G, the circular recess **24m** has a bottom surface **24n.** After the circular recess **24m** is reamed into the bone, a threaded hole **24o** is tapped into the bottom surface **24n** for receiving the threaded screw portion **1012** of the metal anchor **1000A.** Referring to FIG. 42H, next, a plurality of deeper recesses **24p** are reamed or drilled out in an arrangement that match the arrangement of the tabs **1002.** These deeper recesses **24p** provide clearance space for the tabs **1002** that protrude from the base surface **1020** of the metal anchor **1000A** when the metal-backed glenoid implant **1000** is implanted into the bone. Once the recess **24m** is fully prepared as shown in FIG. 42H, because of the threading action that is required to implant the glenoid implant **1000,** the implant procedure involves two steps. First the metal anchor **1000A** is threaded into the recess **24m** until the metal anchor **1000A** is fully seated and the base surface **1020** of the metal anchor **1000A** is in contact with the bottom surface **24n** of the recess **24m.** At this point, the metal anchor **1000A** is seated at the bottom of the recess **24m.** Next, the tabs **1002** of the polymer insert **1000B** are aligned with the cutouts **1013** in the metal anchor **1000A** and the polymer insert **1000B** is pushed into the recess **24m** until the leading ends **1002c** of the tabs **1002** are pushed through the cutouts **1013** and snapped in place. The deeper recesses **24p** provide the clearance for the leading ends **1002c** of the tabs **1002.** Additionally, because the leading ends **1002c** of the tabs **1002** are sitting in the deeper recesses **24p** they prevent the implant **1000** from turning so that the implant **1000** cannot back out by unscrewing.

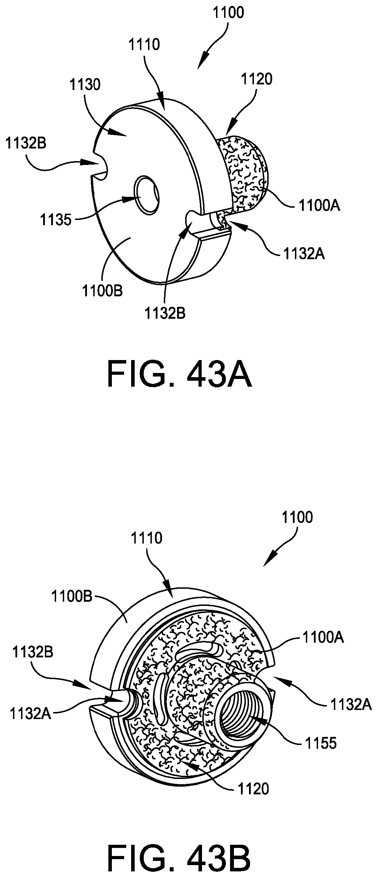

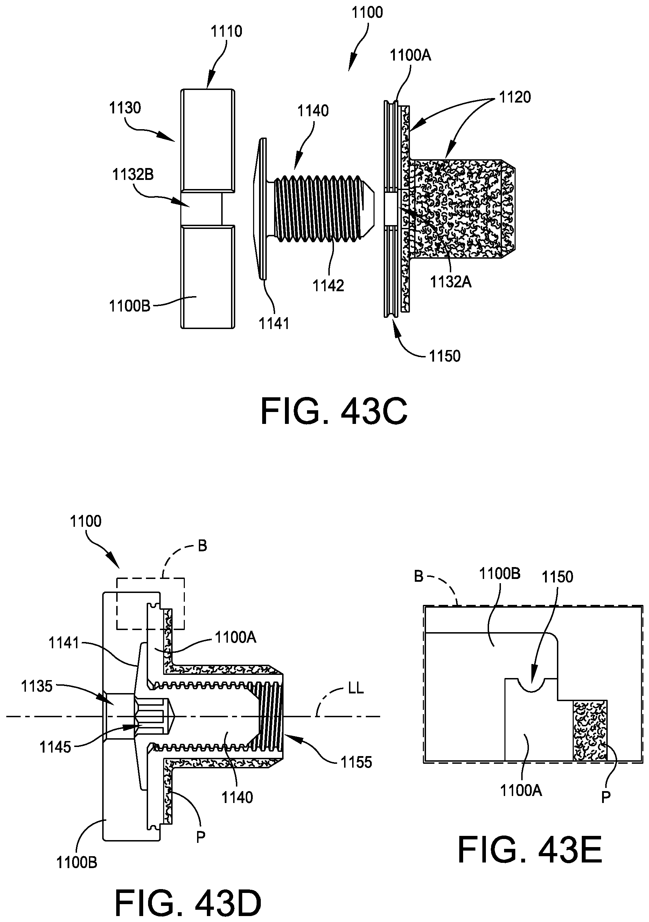

FIGS. 43A-43B show a porous metal-backed glenoid implant embodiment **1100.** The glenoid implant embodiment **1100** comprises a polymer insert **1100B,** providing the articulation surface **1130,** that is overmolded directly onto a metal baseplate **1100A.** The polymer insert **1100B** can be made of high modulus polymer material, such as UHMWPE or PEEK. The articulation surface **1130** for engaging a humeral head (anatomical one or a prosthetic one). In the overmolded structure for the implant **1100** shown in FIGS. 43A-43B, the metal baseplate **1100A** provides the bone-contacting base surface **1120.** In some embodiments, the bone-contacting base surface **1120** is coated with a porous trabecular metal material, such as Wright Medical Technology's ADAPTIS^{™} that can promote bone tissue ingrowth to enhance bonding of the glenoid implant **1100** to glenoid after implantation. FIG. 43C identifies the bone-contacting base surface **1120.** The cross-sectional view of the implant **1100** in FIG. 43D shows the porous trabecular metal coating **P** on the base surface **1120.**

In some embodiments, the side wall **1110** of the polymer insert **1100B** can be tapered for enhanced peripheral fixation.

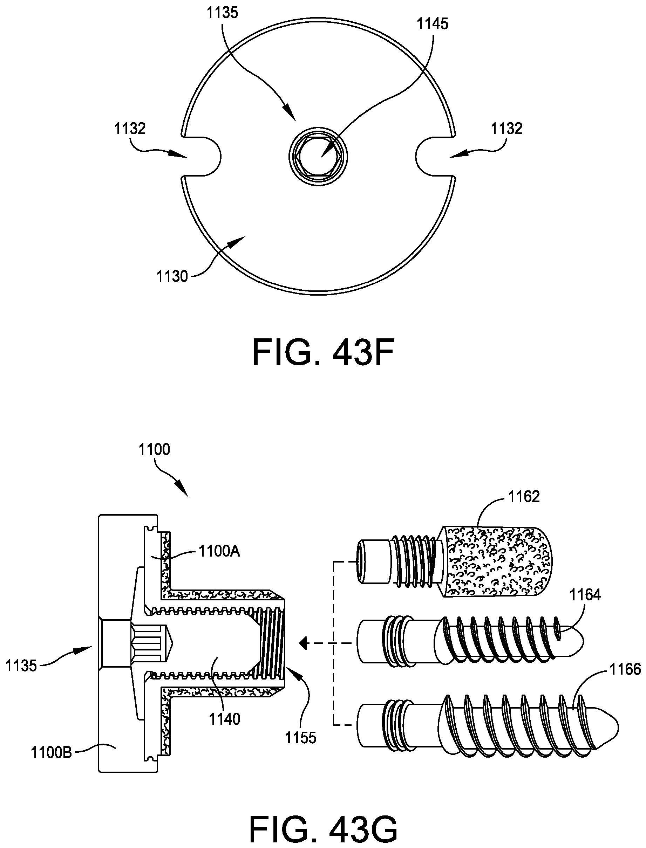

Although the polymer insert **1100B** is overmolded onto the metal baseplate **1100A,** the implant **1100** is an assembly that is configured to be able to remove the polymer insert **1100B** from the metal baseplate **1100A** if necessary. The exploded view of the implant **1100** in FIG. 43C shows that the implant **1100** comprises three components: the baseplate **1100A,** a removal wedge screw **1140,** and the overmolded polymer insert **1100B.** The removal wedge screw **1140** is assembled into the metal baseplate **1100A** before the polymer insert **1100B** is overmolded onto the metal baseplate **1100A.** The removal wedge screw **1140** threads into the threaded hole **1155** that extends through the metal baseplate **1100A** along the longitudinal axis **LL** of the metal baseplate **1100A.** The removal wedge screw **1140** comprises a head portion **1141** and a threaded stem portion **1142.** Then, the polymer insert **1100B** is overmolded over the baseplate **1100A** and the head portion **1141** of the removal wedge screw **1140.** The head portion **1141** has a diameter that covers a substantial portion of the metal baseplate **1100A** such that substantial portion of the surface area of the metal baseplate that is covered by the overmolded polymer insert **1100B** is the head portion **1141.** As will be described below, this configuration allows the removal of the overmolded polymer insert **1100B** using the removal wedge screw **1140.** As shown in the cross-sectional view in FIG. 43D, the removal wedge screw **1140** comprises a tool-engaging socket **1145** at the center of its head portion **1141** that can be used to screw or unscrew the removal wedge screw **1140** with the threaded hole **1155.** The tool-engaging socket **1145** can be configured to mate with one of a variety of known types of screwdrivers. The overmolded polymer insert **1100B** is provided with an access hole **1135** at the center of the insert **1100B** providing access to the tool-engaging socket **1145.** In FIG. 43F, which is a view looking straight on to the articulation surface **1130** of the polymer insert **1100B,** the tool-engaging socket **1145** can be seen through the access hole **1135.** In this example, the tool-engaging socket **1145** is a type that accepts a hexagonal screwdriver tip. By unscrewing the removal wedge screw **1140** out of the threaded hole **1155** of the metal baseplate **1100A,** the head portion **1141** of the removal wedge screw **1140** will lift the overmolded polymer insert **1100B** off from the baseplate **1100A** for removal. To further enable this removal procedure, both the baseplate **1100A** and the polymer insert **1100B** are each configured with a pair of slots **1132A** and **1132B,** respective, that are aligned with each other. These slots **1132A, 1132B** are used for providing counter-torque when removing the removal wedge screw **1140.** When unscrewing the removal wedge screw **1140,** an appropriate tool can be inserted into the pair of slots **1132A, 1132B** to hold the baseplate **1100A** and the polymer insert **1100B** in place and keep them from turning with the removal wedge screw **1140.**

FIG. 43E shows a detailed view of the region **B** in FIG. 43D showing the overmolded polymer insert **1100B** bonding the metal baseplate **1100A.** In some embodiments, to enhance the mechanical integrity of the bonding between the two components, the baseplate **1100A** can comprise a groove **1150** that extend along the periphery of the baseplate **1100A** which results in a more convoluted mating interface between the two components that provides mechanically stronger bonding interface than a straight one, for example.

In some embodiments, different fixation options, such as, modular posts, modular screws, keel, etc. can be used with this porous metal-backed glenoid implant **1100.** For example, a modular post **1162** or a modular screw **1164, 1166** can be threaded into the threaded hole **1155** as illustrated in FIG. 43G.

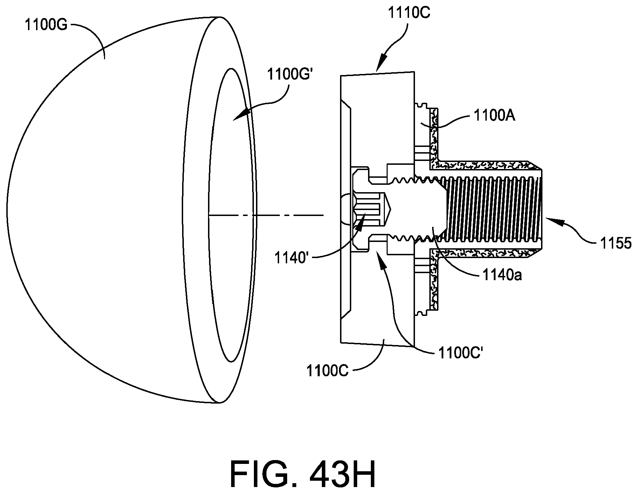

FIG. 43H shows an embodiment where the implant **1100** can be converted to a reverse construct shoulder implant. For this conversion, instead of the polymer insert **1100B,** a taper boss **1100C** that is configured to mate with a glenosphere **1100G** is attached to the metal baseplate **1100A.** In FIG. 43H, the metal baseplate **1100A** and the taper boss **1100C** are shown in cross-section. In some embodiments, the taper boss **1100C** can be configured to be attached to the metal baseplate **1100A** by a screw **1140a.** The screw **1140a** threads into the threaded hole **1155** of the metal baseplate **1100A** as shown. The taper boss **1100C** comprises a hole in the center for receiving the screw **1140a** and the hole in the taper boss **1100C** comprises a ledge **1100C'** that extends inward and catches the head of the screw **1140a.** Thus, the head of the screw **1140a** captures the taper boss **1100C** between the head of the screw and the metal baseplate **1100A** and secures the taper boss **1100C.** The head of the screw is provided with a tool-engaging socket **1140a'** that is configured to mate with one of a variety of known types of screwdrivers. The taper boss **1100C** has a sidewall **1110** that is tapered to engage the glenosphere **1100G** via a Morse taper type locking connection. The glenosphere **1100G** comprises a corresponding female taper surface **1100G'** that engages the male taper surface **1110.** The taper boss **1100C** can also be configured with screw holes (not shown) that align with the slots **1132A** in the metal baseplate **1100A** and can accept screws for enhanced fixation.

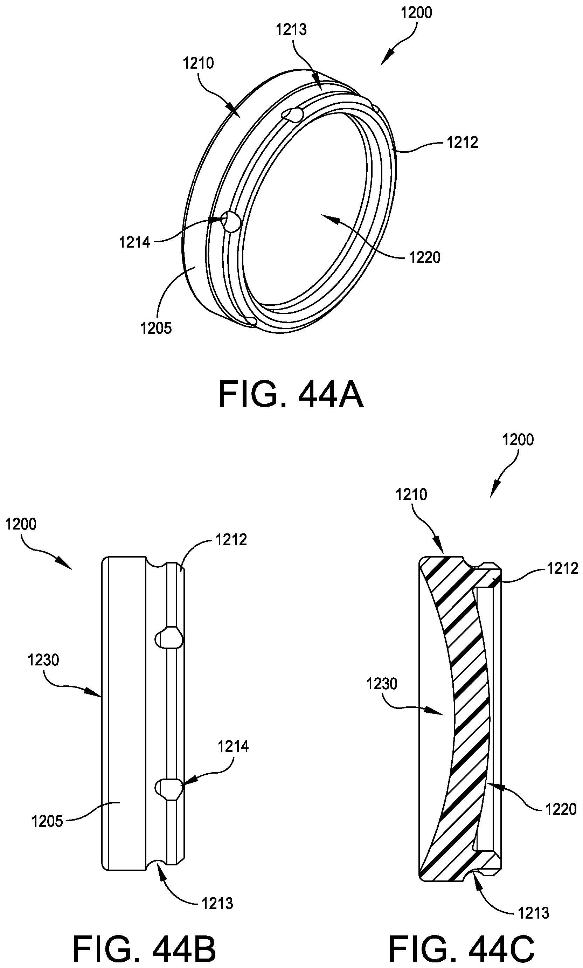

FIGS. 44A-45D show embodiments of glenoid implants employing peripheral ring fixation feature. Referring to FIG. 44A The glenoid implant **1200** comprises a substantially circular body **1205** comprising an articulation surface **1230** on one side and a bone-facing base surface **1220** on the opposite side. Around the periphery of the circular body **1205** is a side wall **1210** that extends between the two surfaces **1220** and **1230.** Extending from the bone-facing base surface **1220** is a peripheral fixation feature that comprises a ring **1212** for engaging a glenoid. As shown in the cross-sectional view in FIG. 44C, in some embodiments, the articulation surface **1230** is contoured to replicate the anatomical articulation surface of the glenoid. In some embodiments, the articulation surface **1230** can have a spherical contour if necessary. The bone-facing base surface **1220** has a spherical contour to engage the glenoid that has been prepared with a complementary surface for receiving the implant **1200.**

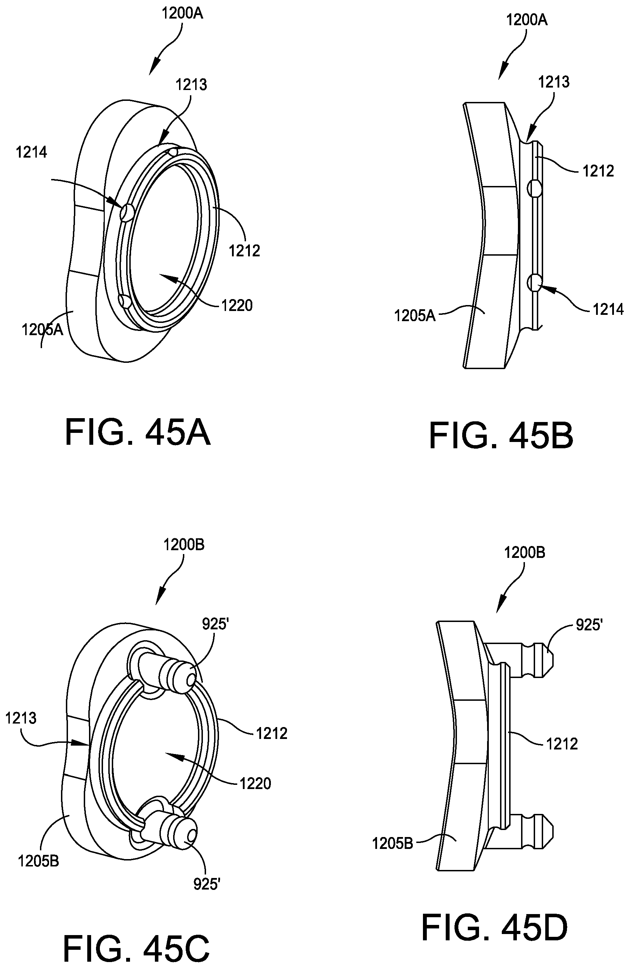

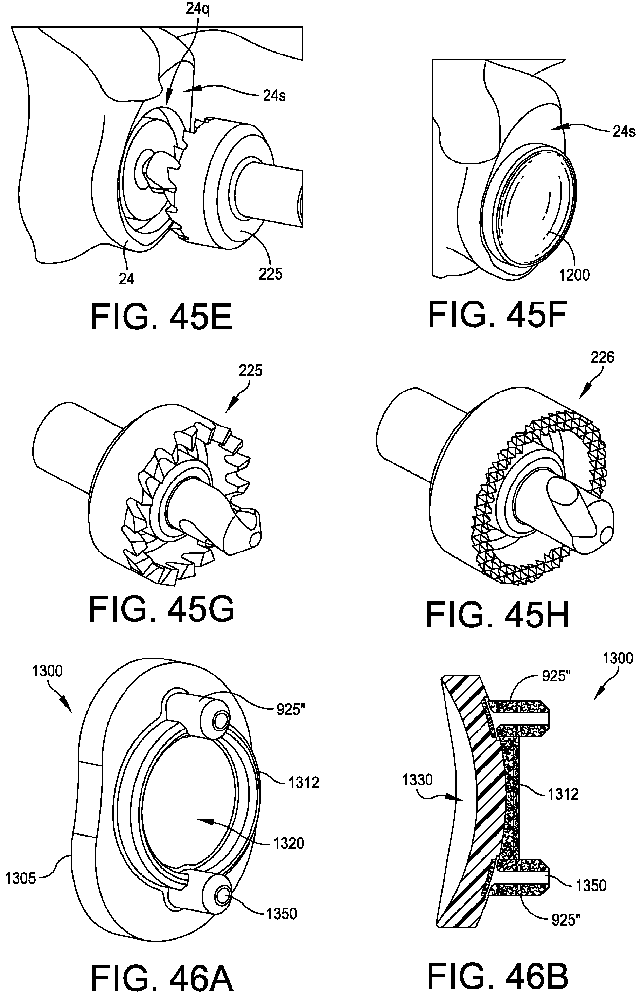

The glenoid implant **1200** further comprises a peripheral ring **1212** that extends from the periphery of the bone-facing surface **1220** and also engages the glenoid which has been prepared with an annular recess **24q** (see FIG. 45E) for receiving the peripheral ring **1212.** In this embodiment **1200** of the glenoid implant where the implant body **1205** has a substantially circular shape, the peripheral ring **1212** is an extension of the side wall **1210.** The peripheral ring **1212** is configured to enhance the quality of fixation to the glenoid. The peripheral ring **1212** comprises a groove **1213** provided on the outer surface of the ring **1212** and extends around the periphery of the ring **1212.** The groove **1213** serves the purpose of holding a quantify of bone cement along the periphery of the ring **1212** to bond to the glenoid. The peripheral ring **1212** can also comprise optional additional plurality of cement grooves **1214** provided along the outer surface of the ring **1212** adjacent to the groove **1213.** The plurality of cement grooves **1214** are oriented axially. In preferred embodiments, the plurality of cement grooves **1214** are located in radially symmetric locations along the ring **1212.** The application of the additional bone cement via the plurality of cement grooves **1214** is intended to prevent rotation of the glenoid implant **1200** after implantation.

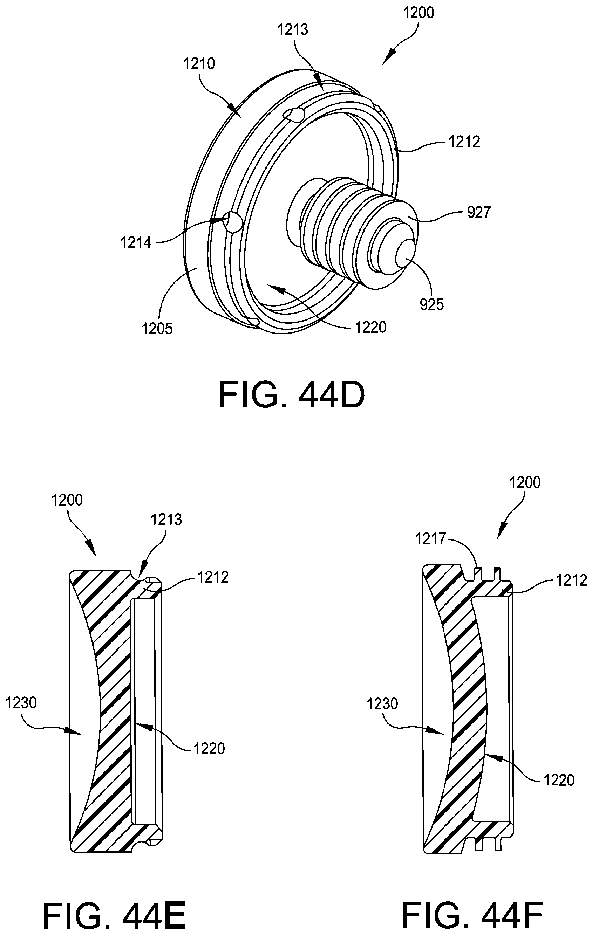

In some embodiments, the glenoid implant **1200** can further comprise one or more additional fixation features extending from the base surface **1220.** These additional fixation features can be any one of the fixation features such as posts, finned anchors, keels, etc. FIG. 44D shows an example of a glenoid implant **1200** comprising a finned anchor **925** extending from the center of the base surface **1220.**

In some other embodiments, the base surface **1220** of the glenoid implant **1200** can be a flat surface as shown in the example cross-section shown in FIG. 44E. In some other embodiments, the outer surface of the peripheral ring **1212** is provided with flexible fins **1217** for cementless application as shown in the example cross-sectional view shown in FIG. 44F. The fins **1217** serve the same function as the fins **927** on the finned anchors **925.**

FIGS. 45A-45B show another glenoid implant **1200A** embodiment that comprises an implant body **1205A** and a peripheral ring **1212** provided on the bone-facing base surface **1220** as a peripheral fixation element similar to the glenoid implant **1200** shown in FIG. 44A. The peripheral ring **1212** for the glenoid implant **1200A** is the same structural features as the peripheral ring **1212** for the glenoid implant **1200,** including all of the optional features. In the embodiment **1200A,** however, the implant body **1205A** is not circular but has a shape that mirrors the outline of an anatomical glenoid. Similar to the glenoid implant **1200,** the glenoid implant **1200A** can also be configured with one or more additional fixation features extending from the base surface **1220.** These additional fixation features can be any one of the fixation features such as posts, finned anchors, keels, etc. FIGS. 45C-45D show a glenoid implant embodiment **1200B** that comprises such additional fixation features. The glenoid implant **1200B** also has a peripheral ring **1212** structure as a peripheral fixation element similar to the glenoid implant **1200** and **1200A.** The peripheral ring **1212** for the glenoid implant **1200B** is the same structural features as the peripheral ring **1212** for the glenoid implants **1200** and **1200A** including all of the optional features. The implant body **1205B** is also shaped to mirror the outline of an anatomical glenoid. In the embodiment **1200B,** however, the implant body **1205B** comprises two or more stabilizing posts **925'** extending from a portion of the peripheral ring **1212** as the one or more additional fixation features mentioned above. The glenoid implant embodiments **1200, 1200A,** and **1200B** can be made of high modulus polymer material, such as UHMWPE or PEEK.

FIG. 45E is an illustration showing how a glenoid **24** may be prepared with an annular recess **24q** for receiving the glenoid implants shown in FIGS. 44A and 45A. The annular recess **24q** is dimensioned to receive the peripheral ring **1212** of the glenoid implants **1200, 1200A.** To receive the implants **1200** or **1200A** that have curved base surface **1220,** the glenoid **24** is first reamed with a curved reamer to prepare the glenoid **24** surface to a curved surface **24s** that matches the curvature of the curved base surface **1220.** Then, a bell saw type reamer **225** is used to ream out the annular recess **24q.**

FIG. 45F is an illustration showing the glenoid implant of FIG. 44A or 45A implanted in the glenoid 24 after the annular recess **24q** has been formed.

FIGS. 45G-45H are illustrations showing examples of bell saw type reamers **225** and 226 that can be used to form the annular recess **24q** in the glenoid **24.**

In addition to the peripheral ring fixation feature, the glenoid implant can have one or more additional non-peripheral fixation features such as posts, finned anchors, or keels.

FIGS. 46A-46B are illustrations showing a glenoid implant example **1300** according to another embodiment. The glenoid implant **1300** comprises an implant body **1305** having an articulation surface **1330** on one side and a bone-facing base surface **1320** on opposing side. The glenoid implant **1300** further comprises a peripheral ring **1312** provided on the bone-facing base surface **1320** as a peripheral fixation element similar to the glenoid implants **1200** and **1200A.** The glenoid implant **1300** also includes one or more posts **925"** exending from the base surface **1320** and located somewhere along the peripheral ring **1312** that can further enhance the implant's fixation with a bone. The

In some preferred embodiments, the one or more posts **925"** are located along the peripheral ring **1312** at radially symmetric positions. The radially spaced posts **925"** along with the peripheral ring **1312** are believed to minimize or substantially eliminate micromotion of the glenoid implant **1300** in a patient.

The peripheral ring **1312** and the posts **925"** are porous trabecular metallic structures that promote bone tissue ingrowth to enhance fixation of the glenoid implant **1300** to glenoid in a cement-less application.

Referring to the cross-sectional view in FIG. 46B, in some embodiments, the posts **925"** are a composite structure having a solid metal core that provides appropriate structural stability (i.e. rigidity) to the posts **925".** The solid metal core can be made of an appropriate alloy that allows the porous trabecular metal coating to bond to the solid metal core and provide lone lifetime of structural reliability in the patient. The combination of the peripheral ring fixation feature **1312** and the one or more additional fixation features **925"** having the solid metal core are referred to herein as having substantially formed of porous trabecular metallic material for promoting bone ingrowth when implanted into a patient.

The glenoid implant **1300** can be made of high modulus polymer material, such as UHMWPE or PEEK. In some embodiments, the implant body **1305** has a shape that mirrors the outline of an anatomical glenoid. The implant body **1305** of high modulus polymer material can be overmolded onto a portion of the metallic peripheral ring **1312** and post **925"** structure. The combination of the trabecular metallic peripheral ring **1312** and the one or more posts **925"** should provide enhanced primary fixation of the glenoid implant **1300** to a glenoid bone.

Further embodiments according to the present disclosure:
1. A glenoid implant comprising:
   a main portion comprising:
   a ring-shaped body defined by a hole extending therethrough, wherein the ring-shaped body comprises an articulation surface provided on one side configured to engage with a humeral head, and
   a base surface provided on an opposite side, configured to be secured into a glenoid.
2. The glenoid implant of Embodiment 1, wherein the hole has a circular shaped outline.
3. The glenoid implant of Embodiment 1, wherein the hole has a polygon shaped outline.
4. The glenoid implant of Embodiment 1, wherein the hole has an irregular shaped outline.
5. The glenoid implant of Embodiment 4, wherein the hole has a patient-specific irregular shaped outline.
6. The glenoid implant of Embodiment 1, wherein the base surface is provided with one or more pegs for securing the implant in a glenoid.
7. The glenoid implant of Embodiment 1, wherein the base surface can be concave, convex, or flat.
8. The glenoid implant of Embodiment 6, wherein each of the one or more pegs comprises one or more slots for accommodating bone cement.
9. The glenoid implant of Embodiment 1, wherein the articulating surface is formed of polyethylene such as ultra-high-molecular-weight polyethylene (UHMWPE), polyether ether ketone (PEEK), or hydrogel.
10. The glenoid implant of Embodiment 1, wherein the main portion comprises at least a portion of its outer surface that is coated with a trabecular metallic material.
11. The glenoid implant of Embodiment 1, wherein the main portion comprises a porous material portion including the base surface; and
   a hydrogel portion that is bonded to the porous material portion and forming the articulation surface opposite from the base surface.
12. A glenoid implant configured to be implanted into a glenoid in an inlay configuration , the glenoid implant comprising:
   a body comprising an articulation surface and an anchoring surface on the opposite side;
   one or more fixation features extending from the anchor surface;
   wherein the body is sized to be smaller than the outline of the glenoid and fits into a recess formed in the glenoid.
13. The glenoid implant of Embodiment 12, wherein the one or more fixation features extending from the anchoring surface can comprise a post, a finned anchor, or a keel.
14. The glenoid implant of Embodiment 13, each of the posts further comprises one or more bone cement pockets.
15. A glenoid implant comprising:
   a circular body comprising an articulation surface and an anchoring surface on the opposite side; and
   an annular side wall extending around the periphery of the circular body, wherein the annular side wall comprises a plurality of retaining legs that are provided along the annular side wall.
16. The glenoid implant of Embodiment 15, wherein the retaining legs are extensions of the annular side wall that are folded over towards the articulation surface and forming a U-shaped leaf spring.
17. The glenoid implant of Embodiment 15, wherein the implant is made of a metal.
18. The glenoid implant of Embodiment 17, wherein the metal is CoCr or titanium.
19. The glenoid implant of Embodiment 15, wherein the implant is made of a high modulus polymer.
20. The glenoid implant of Embodiment 19, wherein the polymer is UHMW polyethylene.
21. The glenoid implant of Embodiment 15, wherein the circular body and the retaining legs are integrally formed from a same material.
22. The glenoid implant of Embodiment 15, wherein the anchoring surface is coated with a porous metallic material.
23. A glenoid implant comprising:
   a circular body comprising an articulation surface and an anchoring surface on the opposite side; and
   an annular side wall extending around the periphery of the circular body, wherein the annular side wall is a fluted surface that comprises a plurality of grooves with blades formed in between two adjacent grooves.
24. The glenoid implant of Embodiment 23, wherein the implant is made of a metal.
25. The glenoid implant of Embodiment 24, wherein the metal is CoCr or titanium.
26. The glenoid implant of Embodiment 23, wherein the anchoring surface is coated with a porous metallic material.
27. A glenoid implant comprising:
   a circular body comprising an articulation surface and an anchoring surface on the opposite side; and
   a plurality of flexible legs extending from the periphery of the circular body toward the direction away from the articulation surface;
   wherein the glenoid implant has a pre-implant configuration and an implanted configuration,
   wherein the glenoid implant can be transformed from the pre-implant configuration to the implanted configuration by plastic deformation of the circular body.
28. The glenoid implant of Embodiment 27, wherein in the pre-implant configuration, the circular body is in a dome-like configuration so that the articulation surface is convex and the anchoring surface is concave; and wherein in the implanted configuration, the circular body is plastically deformed so that the articulation surface is concave and the anchoring surface is convex.
29. The glenoid implant of Embodiment 27, wherein the glenoid implant has longitudinal axis and when in the pre-implant configuration, the flexible legs extend from the periphery of the circular body substantially parallel to or towards the longitudinal axis, and when in the implanted configuration, the flexible legs are radially extending outward.
30. The glenoid implant of Embodiment 27, wherein the implant is made of a metal.
31. The glenoid implant of Embodiment 27, wherein the metal is CoCr, Nitinol, or titanium.
32. The glenoid implant of Embodiment 27, wherein the anchoring surface is coated with a porous metallic material.
33. A glenoid implant comprising:
   a circular body comprising an articulation surface, an anchoring surface on the opposite side, and a side wall extending between the articulation surface and the anchoring surface; and
   at least one peripheral fixation feature that is provided along the periphery of the circular body.
34. The glenoid implant of Embodiment 33, wherein the one or more peripheral fixation feature is a tapered profile of the side wall.
35. The glenoid implant of Embodiment 34, wherein the tapered profile of the side wall is such that the articulation surface is larger in diameter than the anchoring base surface.
36. The glenoid implant of Embodiment 33, wherein the implant is made of a high modulus polymer.
37. The glenoid implant of Embodiment 36, wherein the polymer is UHMW polyethylene.
38. The glenoid implant of Embodiment 33, further comprising one or more fixation features extending from the anchoring surface.
39. The glenoid implant of Embodiment 38, wherein the one or more fixation features extending from the anchoring surface can comprise a post, a finned anchor, or a keel.
40. The glenoid implant of Embodiment 39, wherein the post further comprises one or more bone cement pockets.
41. A glenoid implant comprising:
   a substantially circular body comprising an articulation surface, a base surface on the opposite side of the substantially circular body, and a side wall extending between the articulation surface and the base surface;
   wherein the circular body comprises a plurality of protruding portions that extend radially outward beyond the outer periphery of the substantially circular body; and
   a fixation feature provided on each of the protruding portion and extending from the base surface.
42. The glenoid implant of Embodiment 41, further comprising one or more additional peripheral fixation feature.
43. The glenoid implant of Embodiment 42, wherein the one or more additional peripheral fixation feature is a tapered side wall.
44. The glenoid implant of Embodiment 42, wherein the one or more additional peripheral fixation feature is a protruding lip along the periphery of the base surface.
45. The glenoid implant of Embodiments 41, 43, or 44, further comprising one or more additional non-peripheral fixation features extending from the anchoring surface.
46. The glenoid implant of Embodiment 45, wherein the non-peripheral fixation features can comprise any one of a post, a finned anchor, or a keel.
47. The glenoid implant of Embodiment 41, wherein the base surface is a convex, concave, or flat surface.
48. The glenoid implant of Embodiment 41, wherein the implant is made of a high modulus polymer.
49. The glenoid implant of Embodiment 48, wherein the polymer is UHMW polyethylene.
50. A glenoid implant comprising:
   a substantially circular body comprising an articulation surface, an anchoring surface on the opposite side, and a side wall extending between the articulation surface and the anchoring surface; and
   a locking rim fixation feature that is defined by an annular groove formed into the outer side wall of the substantially circular body and an undercut groove formed into the base surface of the body.
51. The glenoid implant of Embodiment 50, wherein the locking rim has a larger diameter than the substantially circular body and comprises an edge that protrudes radially outward from the body.
52. The glenoid implant of Embodiment 50, further comprising one or more additional non-peripheral fixation features extending from the anchoring surface.
53. The glenoid implant of Embodiment 52, wherein the non-peripheral fixation features can comprise any one of a post, a finned anchor, or a keel.
54. The glenoid implant of Embodiment 50, wherein the implant is made of a high modulus polymer.
55. The glenoid implant of Embodiment 54, wherein the polymer is UHMW polyethylene.
56. A glenoid implant comprising:
   a metal anchor; and
   a polymer insert that are configured to lock into each other.
57. The glenoid implant of Embodiment 56, wherein the metal anchor comprises:
   a circular plate portion comprising two faces and a threaded screw portion extending from the center of one of the two faces that is the bone-facing base surface; and
   a plurality of cutouts along the periphery of the circular plate portion.
58. The glenoid implant of Embodiment 56, wherein the polymer insert comprises an articulation surface and further comprises a plurality of tabs extending from the polymer insert on the side opposite from the articulation surface,
   wherein the plurality of tabs are configured to snap into the corresponding cutouts.
59. The glenoid implant of Embodiment 58, wherein the number and location of the tabs match the number and location of the plurality of cutouts on the plate portion.
60. The glenoid implant of Embodiment 58, wherein each of the tabs comprise one or more compression relieving slots.
61. The glenoid implant of Embodiment 56, wherein the metal anchor is made of titanium.
62. The glenoid implant of Embodiment 56, wherein the polymer insert is made of high modulus polymer material, such as UHMWPE or PEEK.
63. A glenoid implant comprising:
   a metal baseplate ;
   a removal wedge screw threaded into a threaded hole provided at the center of the metal baseplate, wherein the removal wedge screw comprises a threaded shaft portion and a head portion; and
   a polymer insert that is overmolded onto the metal baseplate, wherein the polymer insert comprises an articulation surface, wherein the head portion of the removal wedge screw is captured between the polymer insert and the metal baseplate, wherein unthreading of the removal wedge screw from the metal baseplate in turn removes the polymer insert from the metal baseplate.
64. A glenoid implant comprising:
   a body comprising an articulation surface on one side and a bone-facing base surface on the opposite side;
   a peripheral ring fixation feature that extends from the bone-facing base surface comprises a groove, for holding a quantity of bone cement, provided on the outer surface of the ring and extends around the periphery of the ring.
65. The glenoid implant of Embodiment 64, further comprising one or more additional fixation features extending from the bone-facing base surface.
66. The glenoid implant of Embodiment 65, wherein the one or more additional fixation features can be one of a post, a finned anchor, and a keel.
67. The glenoid implant of Embodiment 64, wherein the base surface is a flat surface.
68. The glenonid implant of Embodiment 64, wherein the body is a circular body.
69. The glenoid implant of Embodiment 64, wherein the body has a non-circular shaped body.
70. A glenoid implant comprising:
   a body comprising an articulation surface on one side and a bone-facing base surface on the opposite side;
   a peripheral ring fixation feature that extends from the bone-facing base surface; and
   one or more additional fixation features extending from the bone-facing base surface, wherein the peripheral ring fixation feature and the one or more additional fixation features are substantially formed of porous trabecular metallic material for promoting bone ingrowth when implanted into a patient.
71. The glenoid implant of Embodiment 70, wherein the one or more additional fixation features are posts having solid metallic cores that provides enhanced structural stability.

Although the devices, kits, systems, and methods have been described in terms of exemplary embodiments, they are not limited thereto. Rather, the appended claims should be construed broadly, to include other variants and embodiments of the devices, kits, systems, and methods, which may be made by those skilled in the art without departing from the scope and range of equivalents of the devices, kits, systems, and methods.

## Claims

1. A glenoid implant (900j, 900k, 900m), comprising:
a body (905) comprising:
an articulation surface (930),
a base surface (920) on the opposite side of the body (905) as the articulation surface (930), and
a side wall (910) extending between the articulation surface (930) and the base surface (920);
an undercut groove (944) formed in the base surface (920) of the body (905);
a peripheral fixation feature (940, 1212) provided along a periphery of the body, the peripheral fixation feature configured with a plurality of cutouts (943, 1214); and
one or more additional fixation features (925, 925', 929) extending from the base surface (920).

2. The glenoid implant of claim 1, comprising a locking rim structure (940) as the peripheral fixation structure, the locking rim structure configured to secure the glenoid implant (900j, 900k, 900m) in a recess prepared in a glenoid, the locking rim structure (940) defined by an annular groove (942) formed in the side wall (910); wherein the plurality of cutouts are a plurality of compression relief cutouts.

3. The glenoid implant of claim 2, wherein the locking rim structure (940) has a diameter that is larger than a diameter of the body (905).

4. The glenoid implant of either claim 2 or claim 3, wherein the locking rim structure (940) includes an edge (941) that protrudes radially outward from the body (905).

5. The glenoid implant of any one of claims 2 to 4, wherein the cutouts (943) divide the locking rim structure (940) into multiple segments.

6. The glenoid implant of any one of the previous claims, wherein the cutouts (943) are located at radially symmetric positions.

7. The glenoid implant of any one of the previous claims, wherein the one or more additional fixation features includes a finned anchor (925).

8. The glenoid implant of claim 7, wherein the finned anchor (925) comprises a substantially constant diameter and a plurality of fins (927) that extend radially outward.

9. The glenoid implant of claim 8, wherein the plurality of fins (927) are spaced apart from each other along a length of the finned anchor (925).

10. The glenoid implant of either claim 8 or claim 9, wherein the plurality of fins (927) are flexible and configured to bend or deform when force is exerted against them.

11. The glenoid implant of any one of claims 7 to 10, wherein a distal end (925d) of the finned anchor (925) includes a tip having a shape that facilitates insertion into a glenoid.

12. The glenoid implant of any one of claims 1 to 6, wherein the one or more additional fixation features includes a plurality of stabilizing posts (925') extending from the base surface (920).

13. The glenoid implant of claim 12, wherein the plurality of stabilizing posts (925') extend substantially perpendicularly from the base surface (920) and include one or a series of indents for accepting bone cement.

14. The glenoid implant of any one of claims 1 to 6, wherein the one or more additional fixation features is a keel (929).

15. The glenoid implant of any one of the previous claims, wherein the glenoid implant is a single unitary structure comprising a polymer material.
